# EUROPÄISCHE PATENTSCHRIFT

(11) **EP 2 498 832 B1**
(45) Veröffentlichungstag und Bekanntmachung des Hinweises auf die Patenterteilung: **16.10.2013**
(21) Anmeldenummer: 10778648.5
(22) Anmeldetag: 12.11.2010
(51) Int. Cl.: A61L 29/06, A61L 29/16

(54) **VERWENDUNG POLYMERER BIOZIDER MATERIALIEN FÜR MEDIZINISCHE ARTIKEL**
USE OF POLYMERIC BIOCIDAL MATERIALS FOR MEDICAL ARTICLES
UTILISATION DE MATÉRIAUX BIOCIDES POLYMÉRIQUES POUR DES ARTICLES MÉDICAUX

(30) Priorität: 12.11.2009 DE 102009052721
(43) Veröffentlichungstag der Anmeldung: 19.09.2012
(73) Patentinhaber: B. Braun Melsungen AG, 34212 Melsungen (DE)
(72) Erfinder: WEIß, André, 34302 Guxhagen (DE); RIEMANN, Thomas, 37247 Großalmerode (DE); SIPPEL, Martin, 34212 Melsungen (DE)
(74) Vertreter: von Kreisler Selting Werner
(86) Internationale Anmeldenummer: PCT/EP2010/067404
(87) Internationale Veröffentlichungsnummer: WO 2011/058144

(56) Entgegenhaltungen:
- WO-A2-2009/009814
- WO-A2-2009/080239
- US-A1- 2002 120 333

## Beschreibung

Die Erfindung betrifft die Verwendung biozider Verknüpfungsprodukte für die Herstellung medizinischer Artikel.

Darüber hinaus betrifft die Erfindung ein Verfahren zur Herstellung eines medizinischen Artikels sowie einen medizinischen Artikel.

Medizinische Artikel bzw. Gegenstände, die in den Körper eines Patienten eingeführt werden, beispielsweise intravasale Katheter, Beatmungsschläuche oder Stents müssen eine möglichst glatte Oberfläche haben, um Beschwerden beim Patienten als auch Ablagerungen auf den Oberflächen zu minimieren. Hergestellt werden diese medizinischen Artikel sowie deren Verpackung oft durch Verfahren der Kunststofftechnik, beispielsweise Formpress-, Spritzguss-, Tiefzieh- und Extrusionsverfahren aus einem Kunststoff, wobei versucht wird, möglichst glatte Oberflächen zu erzielen.

Zur Vermeidung von Infektionen ist es vorteilhaft die medizinischen Artikel mit antimikrobiell wirkenden Mitteln ausgerüstet sein. An die biozide Ausrüstung der medizinischen Artikel werden hohe Anforderungen gestellt, da die Artikel mit Körpergewebe und Körperflüssigkeit in Kontakt kommen. Beispielsweise stellen Katheter, die durch die Hautoberfläche in Arterien oder Venen eingeführt werden, aber auch Wund- oder Thoraxdränageschläuche, häufige Infektionsquellen dar. Insbesondere besteht bei Patienten, die Langzeit-Urinkatheter benötigen, die Gefahr von Harnwegsinfektionen, die zu einer Bakterien- oder einer chronischen Pyelonephritis führen können.

Im medizinischen Bereich spielen insbesondere die zentralen Venenkatheter eine zunehmend größere Rolle bei medizinischen Behandlungen und chirurgischen Eingriffen. Zentrale Venenkatheter werden immer häufiger im Rahmen der Intensivmedizin, aber auch bei Anwendungen, z.B. bei Knochenmarks- und Organtransplantationen, Hämodialyse oder Cardiothoraxchirurgie, eingesetzt.

Ein ähnliches Infektionsrisiko ist aber auch bei allen Vorrichtungen gegeben, welche die Katheter beispielsweise mit Infusionsbehältnissen außerhalb des Körpers verbinden, beispielsweise Verbindungsstücke, T-Stücke, Kupplungen, Filter, Überleitungssysteme, Ventile, Spritzen und Mehrweg-Hähne. All diese Gegenstände werden für die Zwecke dieser Beschreibung und der Patentansprüche als "medizinische Artikel" bezeichnet.

Für die medizinischen Artikel, insbesondere die Katheter, müssen aber nicht nur die hohen Anforderungen an eine glatte Oberfläche beispielsweise zur Vermeidung bzw. Verringerung von Thrombozytenapposition und Biofilmbildung und an die biozide Ausrüstung, die ein Wachsen von Mikroben auf der Oberfläche verhindern oder die Mikroben ganz abtöten sollen, gewährleistet sein, sondern es muss auch sichergestellt werden, dass die biozide Ausrüstung der medizinischen Gegenstände die Materialeigenschaften der medizinischen Artikel nicht negativ beeinflusst. Darüber hinaus muss gewährleistet werden, dass die medizinischen Artikel, insbesondere dann, wenn sie mit Flüssigkeit in Kontakt kommen, einerseits eine hohe biozide Wirksamkeit aufweisen, aber andererseits auch nicht an die Flüssigkeit abgegeben werden, um eine Anreicherung der bioziden Wirkstoffe im Körper zu vermeiden. Die Abgabe eines bioziden Wirkstoffs aus einem medizinischen Artikel bei Flüssigkeitskontakt wird auch als "Leaching" bezeichnet.

Aus der EP 0 229 862 sind medizinische Artikel aus Polyurethan bekannt, auf deren Oberfläche ein antimikrobielles Mittel aufgebracht ist. Aus der EP 0 379 269 und aus der US 2002120333 sind ebenfalls medizinische Artikel, insbesondere Schläuche, bekannt, welche aus einem thermoplastischen Polymer geformt sind und als antimikrobiell wirkendes Mittel Chlorhexidin enthalten. Diese Artikel werden hergestellt, indem zunächst eine Mischung aus Chlorhexidin und Kunststoffgranulate eines thermoplastischen Polymers bereitgestellt wird, die zu einer Schmelze verarbeitet wird, in der das Chlorhexidin gleichmäßig verteilt ist, wobei die Schmelze durch eine Matrix extrudiert wird, um den medizinischen Artikel zu formen. Der Einsatz von bioziden Mitteln auf Basis von Biguaniden, wie beispielsweise Chlorhexidin oder Polyhexamethylenbiguanid, ist jedoch nicht immer zufriedenstellend. Insbesondere besteht ein Verbesserungsbedarf im Hinblick auf die Glattheit der Oberfläche der medizinischen Artikel als auch im Hinblick auf die Reduzierung oder Steuerung des "Leaching"-Effekts.

WO 2009/009814 A2 offenbart einen polymeren Dentalwerkstoff, umfassend einen silikatischen Füllstoff, der mit einem polymeren Guanidinderivat auf Basis eines Alkylendiamins und/oder eines -Oxyalkylendiamins modifiziert ist. Explizit offenbart wird das polymere Guanidinderivat Poly-[2-(2-ethoxy-ethoxyethyl)-guanidium-hydrochlorid], welches jedoch hinsichtlich der antimikrobiellen Wirksamkeit als auch hinsichtlich der Biokompatibilität für Anwendungen von Medizinprodukten der Klasse 3 mit direktem Kontakt zum Blutkreislauf keine zufriedenstellende Ergebnisse lieferte.

Darüber hinaus war es Aufgabe der vorliegenden Erfindung, medizinische Artikel zur Verfügung zu stellen, die mit neuartigen bioziden Materialien ausgerüstet sind, die auch im Hinblick auf eine Resistenz-Entwicklung der Bakterienstämme bezüglich herkömmlicher antimikrobieller Mittel hochwirksam sind.

An biozide Wirkstoffe, die für die Herstellung von medizinischen Artikeln vorgesehen sind, werden hohe Anforderungen gestellt. Zum Einen müssen die bioziden Wirkstoffe unter den Bedingungen der Verarbeitungstechnik, nämlich der thermoplastischen Formgebung, wie Extrusion oder Spritzguss und den dabei auftretenden Temperaturen und Drücken hinreichend erweichen, dürfen sich nicht zersetzen und müssen zudem mit den übrigen Kunststoffbestandteilen des medizinischen Artikel kompatibel sein.

Insbesondere für medizinische Artikel, die im direkten Kontakt mit dem Blutkreislauf stehen, werden zudem hohe Anforderungen an die Biokompatibilität, wobei Faktoren, wie die Zytotoxizität, Hämolyse oder auch allergische Reaktionen eine Rolle spielen, gestellt. Darüber hinaus soll vermieden werden, dass die bioziden Wirkstoffe an das Blut abgegeben werden, es aber gleichsam den medizinischen Artikel mit einer hohen antimikrobiellen Wirksamkeit ausstattet.

Die Aufgabe der vorliegenden Erfindung wird durch die Verwendung neuartiger biozider Verknüpfungsprodukte für die Herstellung medizinischer Artikel gelöst.

Gegenstand der vorliegenden Erfindung ist daher die Verwendung eines bioziden Verknüpfungsprodukts aus
a) einem polymeren oder oligomeren Wirkstoff mit biozider Wirkung, welcher erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
   i) Diamin, das wenigstens einen cycloaliphatischen Rest aufweist, und
   ii) Dialkylentriamin besteht, und
b) einem thermoplastischen Polymer
für die Herstellung medizinischer Artikel.

Ein weiterer Gegenstand ist die Verwendung eines bioziden Verknüpfungsprodukts, welches erhältlich ist durch Reaktion von
a) einem polymeren oder oligomeren Wirkstoff mit biozider Wirkung, welcher erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
   i) Diamin, das wenigstens einen cycloaliphatischen Rest aufweist, und
   ii) Dialkylentriamin besteht,
      mit
b) einem thermoplastischen Polymer,
   für die Herstellung medizinischer Artikel.

Die bioziden Verknüpfungsprodukte sind dadurch gekennzeichnet, dass wenigstens eine kovalente Bindung zwischen dem polymeren oder oligomeren Wirkstoff (Komponente a)) und dem thermoplastischen Polymer (Komponente b)) vorliegt.

Es hat sich gezeigt, dass bei Verwendung der genannten bioziden Verknüpfungsprodukte in Zusammensetzungen, die Kunststoffe, insbesondere thermoplastische Polymere, enthalten, wobei die Kunststoffe für die Herstellung medizinischer Artikel eingesetzt werden, sehr glatte Oberflächen des Kunststoffs gewährleistet werden können, welche sogar jene des Kunststoffs ohne den Zusatz der bioziden Verknüpfungsprodukte übertreffen kann. Zudem kann das biozide Verknüpfungsprodukt als solches, d.h. ohne weitere Zugabe von thermoplastischen Polymeren, direkt für die Herstellung von medizinischen Artikeln verwendet werden, deren Oberflächen eine hervorragende Glätte aufweisen. Neben der hervorragenden bioziden Wirksamkeit, die das biozide Verknüpfungsprodukt als solches aufweist oder den Zusammensetzungen für die medizinischen Artikel verleihen, hat sich auch überraschend herausgestellt, dass das biozide Verknüpfungsprodukt ein äußerst geringes bis gar kein Freietzungsverhalten ("Leaching"-Effekt) aufweist.

Es hat sich zudem überraschend gezeigt, dass die erfindungsgemäß einzusetzenden Verknüpfungsprodukte eine gute Biokompatibilität besitzen, eine hohe antimikrobielle Wirksamkeit gegen eine Vielzahl von Keimen aufweisen und zudem lediglich eine geringe Beeinflussung der thermoplastischen Polymere, die für die Herstellung der medizinischen Artikel benötigt werden, hinsichtlich Festigkeit, Glanz sowie Haltbarkeit zeigen.

### Komponente a) (polymerer oder oligomerer Wirkstoff)

Ein wesentlicher Bestandteil der bioziden Verknüpfungsprodukte ist der polymere oder oligomere Wirkstoff.

Die erfindungsgemäß verwendeten polymeren oder oligomeren Wirkstoffe können sowohl als Homopolymer als auch als Copolymer vorliegen. Vorteilhaft ist dabei, wenn das Guanidin-Säureadditionssalz Guanidiniumhydrochlorid ist. Es sind aber auch weitere Guanidin-Säureadditionssalze, auf Basis anorganischer oder organischer Säuren ebenfalls geeignet. Beispielsweise die Hydroxide, Hydrogensulfate sowie Acetate.

Die erfindungsgemäß verwendeten polymeren oder oligomeren Wirkstoffe liegen bevorzugt als Hydroxidsalz vor. Diese können beispielsweise durch basischen Anionenaustausch aus den entsprechenden Halogeniden, z.B. Chloriden, erhalten werden.

Es hat sich überraschend gezeigt, dass das Verknüpfungsprodukt, wenn es als Hydroxidsalz vorliegt, besonders wirksam ist.

Die polymeren oder oligomeren Wirkstoffe mit biozider Wirkung sind erhältlich durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht.

Die durch die Polykondensation erhältlichen polymeren oder oligomeren Wirkstoffe weisen hierbei bevorzugt eine Polyguanidinstruktur oder, insbesondere im Falle des Einsatzes von Dialkylentriaminen, beispielsweise Diethylentriamin, eine Poly(iminoimidazol)-Struktur auf.

In einer bevorzugten Ausgestaltung der vorliegenden Erfindung umfasst das Amingemisch die Komponente i) (Diamin, das wenigstens einen cycloaliphatischen Rest aufweist) und/oder die Komponente ii) (Dialkylentriamin) in einer Menge von wenigstens 10 mol-%, bevorzugt mindestens 25 mol-%, weiter bevorzugt mindestens 45 mol-%, insbesondere mindestens 85 mol-%, im Speziellen mindestens 95 mol-%, jeweils bezogen auf das gesamte Amingemisch.

Bevorzugt umfasst das Amingemisch zusätzlich ein Alkylendiamin, das besonders bevorzugt eine Verbindung der allgemeinen Formel

NH₂(CH₂)ₙNH₂

in welcher n eine ganze Zahl zwischen 2 und 10, bevorzugt 4 oder 6, ist. Bevorzugt einzusetzende Alkylendiamine tragen endständige Aminogruppen. Speziell bevorzugt ist das Hexamethylendiamin (Hexan-1,6-diamin). Das Alkylendiamin kann in der Polykondensationsreaktion in Mischung mit weiteren Diaminen oder Triaminen, wobei wenigstens ein Amin ausgewählt ist aus der Gruppe, die aus
i) Diamin, das wenigstens einen cycloaliphatischen Rest aufweist, und
ii) Dialkylentriamin besteht,
   bevorzugt ausgewählt aus der Gruppe bestehend aus 4,4'-Methylenbis(cyclohexylamin) und/oder Diethylentriamin unter Ausbildung von Copolymeren eingesetzt werden.

Vorzugsweise kann das Amingemisch weiterhin Oxyalkylendiamine umfassen.

Als Oxyalkylendiamine eignen sich insbesondere solche Oxyalkylendiamine, die endständige Aminogruppen aufweisen. Ein bevorzugtes Oxyalkylendiamin ist eine Verbindung der allgemeinen Formel

NH₂[(CH₂)₂O)]ₙ(CH₂)₂NH₂

in welcher n eine ganze Zahl zwischen 2 und 6, bevorzugt zwischen 2 und 5, weiter bevorzugt zwischen 2 und 4 und insbesondere 2 ist. Bevorzugt sind Polyoxyethylendiamine, im Speziellen Triethylenglycoldiamin. Weiter bevorzugt eingesetzt werden können Polyoxypropylendiamine, insbesondere Di- oder Tripropylenglycoldiamin.

In einer bevorzugten Ausführungsform liegt der polymere oder oligomere Wirkstoff als Homopolymer vor. In diesen Fällen besteht das Amingemisch aus einem Diamin, das wenigstens einen cycloaliphatischen Rest aufweist, oder aus einem Dialkylentriamin.

In einer weiteren bevorzugten Ausführungsform besteht das Amingemisch aus dem Triamin Diethylentriamin. In dieser Variante liegt der polymere oder oligomere Wirkstoff somit als Homopolymer vor, beispielsweise als Poly(iminoimidazol).

In einer weiteren bevorzugten Ausführungsform besteht das Amingemisch aus dem Diamin 4,4'-Methylenbis(cyclohexylamin). Durch Polykondensation mit einem Guanidinsäureadditionssalz entsteht daraus beispielsweise das Homopolymer Poly(4,4'-methylenbis(cyclohexylaminhydrochlorid)).

Besonders bevorzugt sind die polymeren oder oligomeren Wirkstoffe, die erhältlich sind durch Polykondensation eines Guanidinsäureadditionssalzes mit einem Amingemisch, welches wenigstens ein Diamin, das wenigstens einen cycloaliphatischen Rest aufweist, umfasst. Diamine, die wenigstens einen cycloaliphatischen Rest aufweisen, sind beispielsweise cycloaliphatische Diamine, beispielsweise Cyclohexandiamin, Cyclopentandiamin sowie Derivate hiervon. Insbesondere bevorzugt sind solche Diamine, bei denen wenigstens eine NH₂-Gruppe direkt mit dem cycloaliphatischen Rest verknüpft ist. Besonders bevorzugt sind solche Diamine, bei denen beide NH₂-Gruppen jeweils direkt mit ein und demselben cycloaliphatischen Rest oder an verschiedenen cycloaliphatischen Resten verknüpft sind. In einer besonderen Ausführungsform umfasst das Amingemisch 4,4'-Methylenbis(cyclohexylamin).

In einer weiteren Ausführungsform der vorliegenden Erfindung umfasst das Amingemisch wenigstens ein Dialkylentriamin. Die Dialkylentriamine können dabei Alkylenreste unterschiedlicher Kettenlänge aufweisen. Bevorzugt sind allerdings Dialkylentriamine, bei denen die Alkylengruppen die gleiche Länge aufweisen. Bevorzugte Alkylenreste sind dabei Ethylen, Propylen und Butylen sowie Hexylen. In einer besonders bevorzugten Ausführungsform umfasst das Amingemisch das Triamin Diethylentriamin.

In einer weiteren bevorzugten Ausführungsform liegen die erfindungsgemäß verwendeten polymeren oder oligomeren Wirkstoffe in Form von Copolymeren vor. Diese können dabei sowohl willkürlich gemischt als auch als Blockcopolymere vorliegen. Im Falle von Copolymeren enthält das Amingemisch wenigstens zwei verschiedene Amine. Das Amingemisch enthält dabei eine erste Komponente und wenigstens eine zweite Komponente, wobei bevorzugt
a) die erste Komponente ein Diamin oder ein Triamin ist, das ausgewählt ist aus der Gruppe, die aus
   i) Diamin, das wenigstens einen cycloaliphatischen Rest aufweist, und
   ii) Dialkylentriamin besteht, und wobei
b) die zweite Komponente ein Diamin oder ein Triamin ist, das ausgewählt ist aus der Gruppe, die aus
   i) Diamin, das wenigstens einen cycloaliphatischen Rest aufweist,
   ii) Dialkylentriamin,
   iii) Alkylendiamin und
   iv) Oxyalkylendiamin besteht, und
   wobei die erste Komponente von der zweiten Komponente verschieden ist.

Als besonders geeignete copolymere oder cooligomere Wirkstoffe haben sich solche herausgestellt, bei denen die erste Komponente 4,4'-Methylenbis(cyclohexylamin) ist und die zweite Komponente ausgewählt ist aus Diethylentriamin, Hexamethylendiamin und Triethylenglycoldiamin.

In einer weiteren bevorzugten Ausführungsform enthält das copoylmere Guanidinderviat als erste Komponente Diethylentriamin und die zweite Komponente ist ausgewählt aus der Gruppe bestehend aus Hexamethylendiamin und Triethylenglycoldiamin.

Insbesondere bezüglich der bioziden Aktivität und des "Leaching"-Verhaltens bei der Einarbeitung der polymeren oder oligomeren Wirkstoffe in Kunststoffe, insbesondere thermoplastische Polymere, die dann zu medizinischen Artikeln verarbeitet werden, haben sich solche copolymeren Guanidinderivate als besonders geeignet herausgestellt, bei denen mindestens eine Komponente Alkylendiamin, insbesondere Hexamethylendiamin, ist. Im Speziellen bevorzugt sind polymere oder oligomere Wirkstoffe, die erhältlich sind durch Polykondensation eines Guanidinsäureadditionssalzes mit einem Amingemisch, bei dem die erste Komponente Diethylentriamin und/oder 4,4'-Methylenbis(cyclohexylamin) ist und die zweite Komponente Hexamethylendiamin.

Die daraus hergestellten Verknüpfungsprodukte zeigen eine hervorragende biozide Aktivität und ein gutes Leaching-Verhalten.

Speziell bevorzugt sind Amingemische, die Diethylentriamin und Hexamethylendiamin umfassen. Die daraus erhältlichen Wirkstoffe zeigen nicht nur hervorragende antibakterielle Eigenschaften, sondern sind zusätzlich auch biokompatibel, was diese Wirkstoffe insbesondere für medizinische Artikel, die im direkten Kontakt zum Blutkreislauf stehen, eignet.

In einer weiteren Ausführungsform kann die erste Komponente Diamin, das wenigstens einen cycloaliphatischen Rest aufweist, und/oder ein Dialkylentriamin sein und die zweite Komponente ein Oxyalkylendiamin, insbesondere Triethylenglycoldiamin.

Bei der Herstellung von Copolymeren kann das Mischungsverhältnis der einzusetzenden Amine in weiten Bereichen variiert werden. Bevorzugt sind allerdings copolymere oder cooligomere Wirkstoffe, bei denen die Monomere des Amingemischs, also die erste Komponente und die zweite Komponente, in einem Molverhältnis von 4:1 bis 1:4, vorzugsweise 2:1 bis 1:2, vorliegt.

Die erfindungsgemäß einzusetzenden polymeren oder oligomeren Wirkstoffe besitzen bevorzugt ein mittleres Molekulargewicht (Gewichtsmittel) im Bereich von 500 bis 7000, insbesondere von 1000 bis 5000 Dalton.

Die erfindungsgemäß verwendeten polymeren oder oligomeren Wirkstoffe weisen allesamt eine antibakterielle Wirkung auf, die sich mit Hilfe der sogenannten "minimalen Hemmkonzentration" beschreiben lässt. Diese gibt die niedrigste Bakterizidkonzentration an, die das Wachstum von Bakterien in einer bestimmten Lösung hemmt. Besonders günstig ist dabei eine minimale Hemmkonzentration von weniger als 50 µg/ml. Bevorzugt weisen die erfindungsgemäß zu verwendenden polymeren polymeren oder oligomeren Wirkstoffe eine minimale Hemmkonzentration von weniger als 10 µg/ml, insbesondere von weniger als 5 µg/ml auf. Je geringer diese Konzentration ist, umso effektiver kann der entsprechende polymere oder oligomere Wirkstoff als Biozid eingesetzt werden und die Biozidie des Verknüpfungsprodukts entsprechend erhöhen.

In einer bevorzugten Ausführungsform weisen die erfindungsgemäß einzusetzenden polymeren oder oligomeren Wirkstoffe eine minimale Hemmkonzentration von 50 µg/ml oder weniger, bevorzugt 30 µg/ml oder weniger, besonders bevorzugt 10 µg/ml oder weniger und insbesondere 5 µg/ml oder weniger auf.

Die erfindungsgemäß einzusetzenden polymeren oder oligomeren Wirkstoffe lassen sich relativ einfach herstellen. Die Polykondensation kann durch Mischen eines Äquivalents eines Säureadditionssalzes mit einem Äquivalent der Aminmischung und anschließendes Erhitzen, vorzugsweise in einem Bereich von 140 bis 180 °C und Rühren der Schmelze bei erhöhten Temperaturen, vorzugsweise in einem Bereich von 140 bis 180 °C, bis die Gasentwicklung beendet ist, erfolgen. Die Polykondensation erfolgt regelmäßig in einem Zeitraum von mehreren Stunden, bei der die Schmelze bevorzugt in dem Temperaturbereich von 140 bis 180 °C gerührt wird. Ein bevorzugter Reaktionszeitraum ist 1 bis 15 Stunden, vorzugsweise 5 bis 10 Stunden.

Je nach gewünschtem Endprodukt variiert das Verfahren leicht, so ist es zur Herstellung des Homopolymeren auf Basis von 4,4'-Methylenbis(cyclohexylamin) günstig, wenn die Reaktionstemperatur 170 °C beträgt. Das auf Basis von Diethylentriamin hergestellte Homopolymer kann dagegen bei 150 °C gewonnen werden. Die Herstellung der erfindungsgemäß einzusetzenden copolymeren Wirkstoffe erfolgt wiederum bevorzugt in einem Temperaturbereich von etwa 170 °C.

Es hat sich zudem überraschend gezeigt, dass beispielsweise bei der Kondensation von Triaminen cyclische Strukturen, beispielsweise Iminoimidazol-Strukturen, innerhalb der Polymereinheiten entstehen können, so dass es sich bei den erfindungsgemäß einzusetzenden Wirkstoffen nicht nur um polymere oder oligomere Guanidinderivate, sondern auch um polymere oder oligomere Iminoimidazolderivate handeln kann.

Insbesondere ist es vorteilhaft, wenn der polymere oder oligomere Wirkstoff eine Struktur aufweist, die aus der Gruppe umfassend 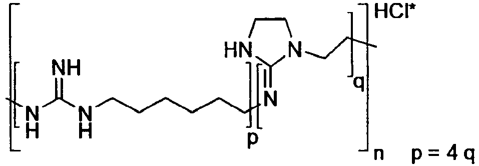 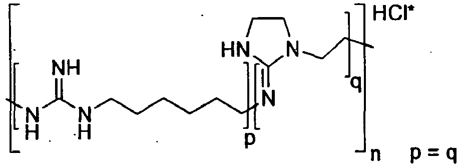 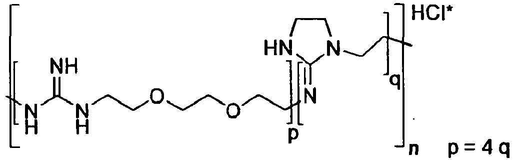 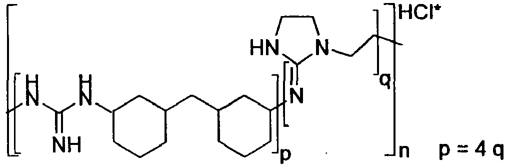 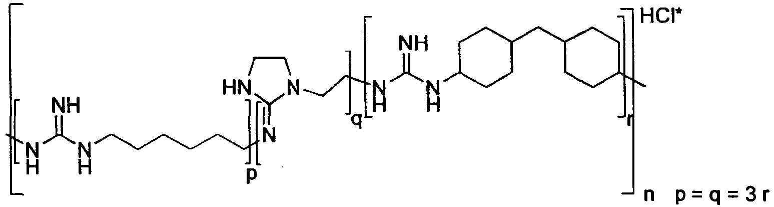 ausgewählt ist, wobei
HCl* bedeutet, dass das HCl nicht kovalent gebunden ist,
n ist eine natürliche Zahl, vorzugsweise von 1 bis 20, weiter bevorzugt von 2 bis 16 und insbesondere von 3 bis 8,
p, q und r sind ganze Zahlen, die das bevorzugte molare Verhältnis der Strukturfragmente zueinander in den Formeln definieren.

In einer bevorzugten Ausführungsform der vorliegenden Erfindung sind die polymeren oder oligomeren Wirkstoffe erhältlich durch Polykondensation eines Guanidinsäureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht,
und wobei die Mischung aus Guanidinsäureadditionssalz und Amingemisch auf eine Temperatur oberhalb von 140 °C, vorzugsweise in einem Bereich von 150 bis 170 °C, erwärmt wird und die Reaktionsmischung bei dieser Temperatur für wenigstens 1 Stunde, vorzugsweise wenigstens 5 Stunden, gehalten wird.

### Komponente b) (thermoplastische Polymer)

Eine weitere wesentliche Komponente des bioziden Verknüpfungsprodukts ist ein thermoplastische Polymer. Bevorzugt sind thermoplastische Polymere, die im Rahmen eines "reactive processing" mit den polymeren oder oligomeren Wirkstoffen (Komponente a)) eine kovalente Verknüpfung eingehen. Hierfür geeignete thermoplastische Polymere sind beispielsweise Polymere, die durch Polykondensation oder Polyaddition erhältlich sind.

Das thermoplastische Polymer ist bevorzugt ausgewählt aus der Gruppe bestehend aus Polyurethan, Polycarbonat, Polyethersulfon, Silikon, Polyester, Polyamid, Polycaprolacton, Polylactid und Polyharnstoff, insbesondere Polykondensate.

Besonders bevorzugt sind Polyethylenterephthalat, Polybutylenterephthalat, Polyamid PA6, PA66, PA610, PA11, PA12, aliphatische und aromatische Polycarbonate sowie aliphatische und aromatische Polyurethane

### Verknüpfungsprodukt

Die erfindungsgemäß für die Herstellung der medizinischen Artikel vorgesehenen bioziden Verknüpfungsprodukte umfassen die Verknüpfung aus den zuvor beschriebenen polymeren oder oligomeren Wirkstoffen (Komponente a)) mit einem thermoplastischen Polymer (Komponente b)), das heißt, Wirkstoff und Thermoplast sind kovalent miteinander verbunden.

Die Verknüpfungsprodukte sind erhältlich durch chemische Reaktion der zuvor genannten Komponente a) mit der Komponente b) unter Ausbildung mindestens einer kovalenten Bindung zwischen Komponente a) und Komponente b).

Bei der Herstellung der erfindungsgemäß zu verwendenden bioziden Verknüpfungsprodukte erfolgt die Verknüpfung des polymeren Guanidinderivats mit dem thermoplastischen Polymer bevorzugt unter den Bedingungen des "reactive processing". Hierzu wird der polymere oder oligomere Wirkstoff (Komponente a)) mit dem thermoplastischen Polymer (Komponente b)) vermischt und die Reaktionsbedingungen so gewählt, dass eine kovalente Anbindung der Komponente a) an das thermoplastische Polymer (Komponente b)) erfolgt. Dies kann beispielsweise dadurch geschehen, dass das thermoplastische Polymer so ausgewählt wird, dass es noch Reaktivgruppen trägt, beispielsweise Isocyanatgruppen.

Die Anbindung des polymeren Guanidinderivats kann jedoch auch unter ausgewählten Bedingungen im Rahmen einer Umkondensation erfolgen. Besonders geeignete thermoplastische Polymere sind hierfür aliphatische Polyurethane und/oder aromatische und/oder araliphatische Polyurethane. Bevorzugt wird der oligomere oder polymere Wirkstoff in flüssiger Form, vorzugsweise gelöst in einem Lösungsmittel, mit dem thermoplastischen Polymer zur Reaktion gebracht wird. Es hat sich überraschend gezeigt, dass insbesondere unter diesen Bedingungen eine kovalente Anbindung vereinfacht erfolgt. Geeignete Lösungsmittel sind beispielsweise polare Lösungsmittel wie Alkohole, hervorragend eignet sich jedoch auch Wasser.

In einer besonders bevorzugten Ausgestaltung der vorliegenden Erfindung wird daher der oligomere oder polymere Wirkstoff in Form einer wässrigen Lösung, bevorzugt einer mindestens 20 Gew.-%igen, weiter bevorzugt 20 bis 80 Gew.-%igen, besonders bevorzugt 25 bis 50 Gew.-%igen wässrigen Lösung, mit dem thermoplastischen Polymer zur Reaktion gebracht.

Im Rahmen des reactive processing kann die Reaktion zwischen Komponente a) und Komponente b) auch während der Verarbeitung erfolgen. Bevorzugt erfolgt die Reaktion durch Vermischen in einem Extruder.

Das biozide Verknüpfungsprodukt ist bevorzugt erhältlich, wenn die Reaktion zwischen Komponente a) und Komponente b) bei einer Temperatur von mehr als 120°C, bevorzugt mehr als 140°C, besonders bevorzugt von mehr als 160°C und insbesondere zwischen 160°C und 300°C erfolgt.

Es hat sich überraschend herausgestellt, dass das biozide Verknüpfungsprodukt insbesondere dann erhältlich ist, wenn das thermoplastische Polymer bei

Temperaturen oberhalb von 120°C, bevorzugt oberhalb von 160°C schmelzextrudiert wird und eine wässrige Lösung, vorzugsweise eine 20 bis 50 Gew.-%ige wässrige Lösung des polymeren Guanidinderivats zugegeben wird. Gerade unter diesen Reaktionsbedingungen konnte die kovalente Anbindung der polymeren Guanidinderivate an das thermoplastische Polymer beobachtet werden.

Besonders geeignet für die kovalente Anbindung, insbesondere im Rahmen einer Schmelzextrusion, sind Polyester, Polylactide, Polycaprolactone, Polyamide, Polyesteramide, Polycarbonate, Polyurethane und Polyharnstoffe.

Besonders bevorzugt sind Polyethylenterephthalat, Polybutylenterephthalat, Polyamid PA6, PA66, PA610, PA11, PA12, aliphatische und aromatische Polycarbonate sowie aliphatische und aromatische Polyurethane.

Besonders bevorzugt sind Verknüpfungsprodukte, bei denen der polymere oder oligomere Wirkstoff kovalent an das thermoplastische Polymer gebunden ist, wobei bevorzugt das thermoplastische Polymer aus der Gruppe umfassend thermoplastische aliphatische und aliphatische/aromatische Polyurethane, aliphatische und aliphatische/aromatische Polyester, aliphatische und aliphatische/aromatische Polyamide, aliphatische und aliphatische/aromatische Polycarbonate, aliphatische und aliphatische/aromatische Polyharnstoffe, aliphatische und aliphatische/aromatische Polyesteramide ausgewählt ist und wobei der polymere oder oligomere Wirkstoff cyclische Strukturen in der Hauptkette aufweist.

Insbesondere ist des vorteilhaft, wenn der polymere oder oligomere Wirkstoff eine Struktur aufweist, die aus der Gruppe umfassend 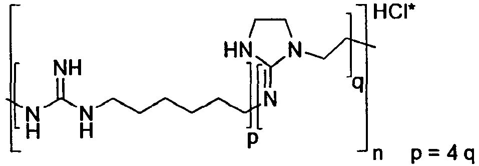 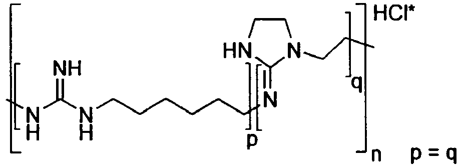 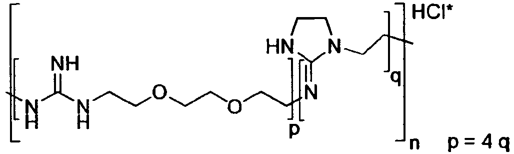 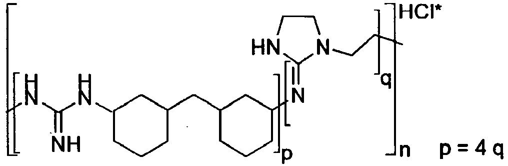 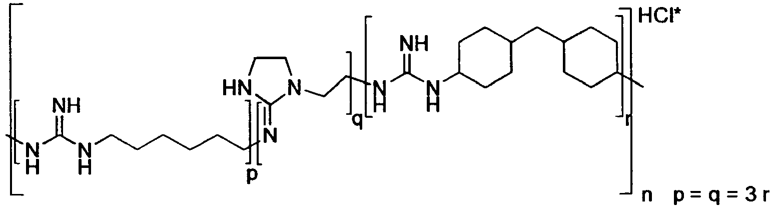 ausgewählt ist, wobei
HCl* bedeutet, dass das HCl nicht kovalent gebunden ist,
n ist eine natürliche Zahl, vorzugsweise von 1 bis 20, weiter bevorzugt von 2 bis 16 und insbesondere von 3 bis 8,
p, q und r sind ganze Zahlen, die das bevorzugte molare Verhältnis der Strukturfragmente zueinander in den Formeln definieren.

Die Verknüpfungsprodukte sind bevorzugt erhältlich durch Compoundierung des polymeren oder oligomeren Wirkstoffs mit dem thermoplastischen Polymer unter Bedingungen des reactive processing. Dies geschieht insbesondere dann, wenn das thermoplastische Polymer ein thermoplastisches aliphatisches Polyurethan (TAPU) oder ein thermoplastisches aliphatisches/aromatisches Polyurethan (TAAPU) ist und wenn der polymere oder oligomere Wirkstoff eine cyclische Struktur aufweist, die ausgewählt ist aus der Gruppe, umfassend 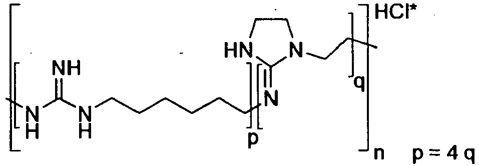 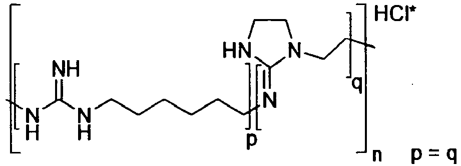 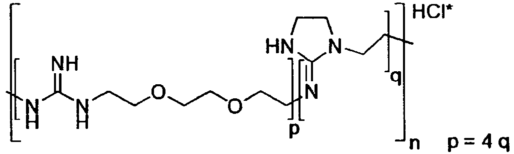 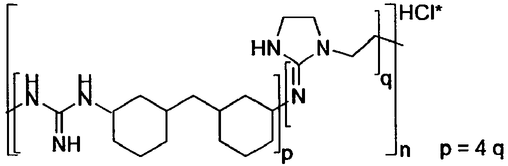 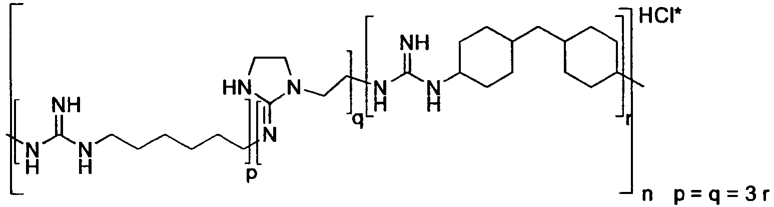 wobei
HCl* bedeutet, dass das HCl nicht kovalent gebunden ist,
n ist eine natürliche Zahl, vorzugsweise von 1 bis 20, weiter bevorzugt von 2 bis 16 und insbesondere von 3 bis 8,
p, q und r sind ganze Zahlen, die das bevorzugte molare Verhältnis der Strukturfragmente zueinander in den Formeln definieren.

Erfindungsgemäß werden die bioziden Verknüpfungsprodukte als solches für die Herstellung von medizinischen Artikel verwendet oder in Zusammensetzungen für medizinische Artikel eingesetzt. Je nach biozider Wirksamkeit bioziden Verknüpfungsprodukte sowie der Art und des Aufbaus des medizinischen Artikels können die Zusammensetzungen für den medizinischen Artikel in einer bevorzugten Ausführungsform den polymeren oder oligomeren Wirkstoff in einer Menge von maximal 10,0 Gew.-%, bevorzugt in einer Menge von 0,01 bis 5 Gew.-% und insbesondere in einer Menge von 1,0 bis 4,0 Gew.-%, jeweils bezogen auf die Zusammensetzung für den medizinischen Artikel, enthalten.

Im Rahmen der vorliegenden Erfindung liegen die Verknüpfungsprodukte bevorzugt in Form ihrer Halogenid-Salze (Säureadditionssalze), insbesonder als HCl-Additionssalz vor. Weiter bevorzugt können die Verknüpfungsprodukte als Hydroxide vorliegen. Diese zeigen eine besonders gute Biozidie und können beispielsweise aus den Chloriden durch basischen Anionentausch erhalten werden.

Ein besonderer Vorteil der erfindungsgemäß einzusetzenden bioziden Verknüpfungsprodukte ist deren Einarbeitbarkeit in Kunststoffen, insbesondere thermoplastischen Polymeren, die häufig den wesentlichen Bestandteil von Zusammensetzungen für medizinische Artikel bilden. Es hat sich überraschend gezeigt, dass sich die bioziden Verknüpfungsprodukte nicht nur problemlos in Kunstoffen, insbesondere thermoplastischen Polymerzusammensetzungen einarbeiten lassen, sondern dass darüber hinaus die mechanischen Eigenschaften, wie beispielsweise die Zugbelastbarkeit oder der Biegewiderstand, nur unwesentlich beeinflusst werden. Darüber hinaus zeigte sich überraschend, dass der Einsatz der bioziden Verknüpfungsprodukte in Zusammensetzungen umfassend Kunststoffe, insbesondere thermoplastische Polymere, für medizinische Artikel bei der Verarbeitung zu extrem glatten Oberflächen führt und darüber hinaus einen steuerbaren "Leaching"-Effekt aufweist. So können die bioziden polymeren oder oligomeren Wirkstoffe so verarbeitet werden, dass sie nicht von Flüssigkeiten wie beispielsweise Wasser oder Ethanol aus dem Polymer-Blend freigesetzt werden, was beispielsweise für medizinische Artikel wie Katheter von Bedeutung ist. Andererseits kann eine gezielte Freisetzung auch wünschenswert sein, beispielsweise bei Wundauflagen. Für diesen Fall ist es möglich den Zusammensetzungen für die Herstellung der medizinischen Artikel zusätzlich die zuvor definierten polymeren oder oligomeren Wirkstoffe (Komponente a)) zuzugeben. Die mit den bioziden Verknüpfungsprodukten ausgerüsteten Zusammensetzungen, insbesondere Zusammensetzungen umfassend thermoplastische Polymere für medizinische Artikel, weisen eine hervorragende antimikrobielle Wirksamkeit auf, obschon sie wie im Falle der Katheteranwendungen keinen Leaching-Effekt aufweisen.

In einer bevorzugten Ausführungsform umfasst die Zusammensetzung für medizinische Artikel neben den erfindungsgemäß zu verwendenen Verknüpfungsprodukten weiterhin Kunstoffe, vorzugsweise thermoplastische Polymere, insbesondere ausgewählt aus Polyurethan, Polyolefin, Polyvinylchlorid, Polycarbonat, Polystyrol, Polyethersulfon, Silikon und Polyamid. Besonders bevorzugt umfassen die Zusammensetzungen für medizinische Artikel Polyurethan, Polyethylen oder Polypropylen.

Darüber hinaus können die Zusammensetzungen zusätzlich die oben definierte Komponente a) in nicht gebundener Form aufweisen.

Darüber hinaus können die Zusammensetzungen für medizinische Artikel weitere übliche Zusatzstoffe enthalten. Hier kommen insbesondere unter physiologischen Bedingungen inerte Füllstoffe in Frage. Besonders geeignet ist Bariumsulfat. Beispielsweise kann ein geeignetes BaSO₄ unter dem Handelsnamen Blancfix^{®} von der Firma Sachtleben Chemie GmbH bezogen werden. Die Füllstoffe liegen bevorzugt in den Zusammensetzungen für medizinische Artikel in einer Menge von 10 bis 35 Gew.-%, bezogen auf die Gesamtmischung, vor. Vorteilhafterweise weisen die Füllstoffe eine mittlere Teilchengröße von 0,01 µm bis 10 µm auf.

In einer bevorzugten Ausgestaltung der vorliegenden Erfindung ist die Zusammensetzung jedoch im Wesentlichen frei von silikatischen Füllstoffen, da diese die Oberflächenglätte als auch den Leachingeffekt negativ beeinflussen können.

Im Wesentlichen frei im Rahmen der vorliegenden Erfindung bedeutet, dass die silikatischen Füllstoffe in einer Menge unterhalb von 1 Gew.-%, bevorzugt unterhalb von 0,5 Gew.-%, weiter bevorzugt unterhalb von 0,01 Gew.-% und insbesondere frei von jeglichen silikatischen Füllstoffen, vorliegen können, wobei sich die Gewichtsangaben auf das Gesamtgewicht der Zusammensetzung für die Herstellung des medizinischen Artikels bezieht.

Medizinische Artikel im Sinne der vorliegenden Erfindung sind insbesondere ausgewählt aus der Gruppe bestehend aus zentralnervösen Kathetern; peripheren Venenkathetern; Beatmungsschläuche; Stents; Produkten für Anwendung in der Regionalanästhesie, insbesondere Katheter, Kuppelungen, Filter; Produkten für die Infusionstherapie, insbesondere Behältnisse, Ports, Überleitungssysteme, Filter; Zubehör wie Konnektoren, Spikes, Ventile, Dreiwegehähne, Spritzen, Leitungen, Zuspritzports; Produkte der Zubereitung, insbesondere Transfersets, Mixsets; urologische Produkte, insbesondere Katheter, Urinmess- und -sammelgeräte; Wunddrainage; Wundversorgung; chirurgische Nahtmaterialien; Implantationshilfsstoffe sowie Implantate, insbesondere Kunststoffimplantate, beispielsweise Herniennetze, Vliese, Gestricke, Gewirke, Ports, Portkatheter, Gefäßprothesen; Desinfektionsmittel; chirurgisches Einmalinstrumentarium; Thoraxdrainagen; Sonden; Katheter; Gehäuse von medizinischen Geräten, insbesondere Infusionspumpen, Dialysegeräte und Monitore; künstliche Gebisse; Behälter für Flüssigkeiten, insbesondere Kontaktlinsenbehälter.

Medizinische Artikel im Rahmen der vorliegenden Erfindung erfassen auch Zubehörteile für medizinische Produkte, wie beispielsweise Spritzgussteile und Formteile. Eine besondere Bedeutung kommt der Verwendung der polymeren oder oligomeren Wirkstoffe als Additiv in Beschichtungen für chirurgisches Nahtmaterial zu.

Ein bevorzugter medizinischer Artikel im Rahmen der vorliegenden Erfindung ist eine Wundauflage.

Besonders bevorzugte medizinische Artikel sind schlauchförmige medizinische Artikel. Solche Artikel weisen zumindest einen schlauchförmigen Bestandteil auf.

Schlauchförmige medizinische Artikel im Sinne der vorliegenden Erfindung sind solche medizinischen Artikel, die Fluide führen können. Insbesondere sind die medizinischen Artikel ausgewählt aus der Gruppe bestehend aus Katheder; zentralvenösen Kathetern; peripheren Venenkathetern; Beatmungsschläuche; Stents; Kuppelungen; Ports; Überleitungssysteme; Konnektoren, Spikes, Ventile, Dreiwegehähne, Spritzen, Leitungen, Zuspritzports; Wunddrainage; Thoraxdrainagen und Sonden.

Besonders bevorzugte medizinische Artikel sind Katheter, insbesondere solche, die durch Extrusion von Zusammensetzungen umfassend Polyurethan und/oder Polyethylen und/oder Polyamid hergestellt werden.

Ein besonders geeignetes Polyamid ist unter dem Handelsnamen Pebax^{®} (Arkema) erhältlich. Es handelt sich dabei um ein Polyamid, das Polyether-Blöcke aufweist.

Aufgrund ihrer hervorragenden antimikrobiellen Wirkung sind die polymeren oder oligomeren Wirkstoffe auch als Additiv für Reinigungsmittel oder Desinfektionsmittel, insbesondere Händedesinfektionsmittel geeignet.

Ein weiterer Gegenstand der vorliegenden Erfindung ist ein Verfahren zur Herstellung eines medizinischen Artikels, vorzugsweise eines schlauchförmigen medizinischen Artikels, umfassend die folgenden Schritte:
a) Zusammenführen und Vermischen des bioziden Verknüpfungsprodukts, wie zuvor definiert, mit gegebenenfalls einem thermoplastischen Polymer, und
b) Einsetzen des unter a) erhaltenen Gemisches in einem oder mehreren Formgebungsverfahren unter Ausbildung eines medizinischen Artikels, vorzugsweise eines schlauchförmigen medizinischen Artikels.

Bevorzugte biozide Verknüpfungsprodukte und bevorzugte thermoplastische Polymere entsprechen den zuvor genannten.

Das Vermischen in Schritt a) erfolgt bevorzugt durch Schmelzkneten. Gegebenfalls können die Verfahrensbedingungen auch so gewählt werden, dass im Rahmen eines reactive processing, das Verknüpfungsprodukt aus Komponente a) und Komponente b) erst während des Herstellverfahrens für den medizinischen Artikel entsteht. Die Komponente a) könnte dazu beispielsweise als wässrige Lösung dem geschmolzenen thermoplastischen Polymer zugegeben werden und anschließend in einem Extruder vermischt werden. Bevorzugt erfolgt das Schmelzkneten bei Temperaturen oberhalb von 100°C, weiter bevorzugt oberhalb von 150°C.

Bevorzugt wird das biozide Verknüpfungsprodukte dem Formgebungsverfahren in Schritt b) als Granulat oder Masterbatch zugeführt. Die Herstellung von Granulaten kann nach dem Fachmann auf dem Gebiet der Kunststofftechnologie geläufigen Verfahren erfolgen. Bevorzugt ist der Masterbatch ein Granulat, das biozide Verknüpfungsprodukt in einer höheren Konzentration als der vorgesehenen Endkonzentration im medizinischen Artikel aufweist. Bei Einsatz eines Masterbatches wird dieser daher durch Zugabe von weiterem Kunststoff weiter auf die gewünschte Endkonzentration verdünnt.

Das in Schritt b) des erfindungsgemäßen Verfahrens zum Einsatz kommende Formgebungsverfahren ist bevorzugt ein Extrusionsverfahren oder Spritzgussverfahren. Hiermit können beispielsweise schlauchartige Komponenten des Katheters hergestellt werden.

Die Formgebungsverfahren werden vorzugsweise bei Temperaturen oberhalb des Schmelzpunkts des in Schritt a) hergestellten Gemisches durchgeführt, besonders bevorzugt in einem Temperaturbereich oberhalb von 160 °C, weiter bevorzugt in einem Bereich von 180 - 260 °C.

Ein weiterer Gegenstand der vorliegenden Erfindung ist ein medizinischer Artikel umfassend ein biozides Verknüpfungsprodukt wie zuvor definiert.

Bevorzugte Verknüpfungsprodukte und bevorzugte Kunststoffe entsprechen den zuvor genannten.

Das in dem erfindungsgemäßen medizinischen Artikel sowie in dem erfindungsgemäßen Verfahren einzusetzende thermoplastische Polymer oder auch die Komponente b) des Verknüpfungsprodukts ist bevorzugt ein thermoplastisches Polyurethan. Es haben sich hier als besonders geeignet Polyurethane herausgestellt, die aus einer Kombination von 4,4'-Diphenylmethan-diisocyanat (MDI) und einem Polyol auf Polyester- oder Polyetherbasis bestehen. Vorteilhafterweise umfasst das Polyol einen Polytetramethylenglycolether. Weitere geeignete thermoplastische Polymere, die bevorzugt für Zusammensetzungen der erfindungsgemäßen medizinischen Artikel in Frage kommen, sind beispielsweise ausgewählt aus Polyurethan, Polyolefin, Polyvinylchlorid, Polycarbonat, Polystyrol, Polyethersulfon, Silikon und Polyamid. Besonders bevorzugt umfassen die Zusammensetzungen für medizinische Artikel Polyurethan, Polyethylen oder Polypropylen oder Polyamid.

Bevorzugte medizinische Artikel entsprechen den zuvor genannten. Insbesondere bevorzugte medizinische Artikel sind Wundauflagen und Katheter.

Weitere Merkmale, Einzelheiten und Vorteile der Erfindung ergeben sich aus dem Wortlaut der Ansprüche sowie aus der folgenden Beschreibung von Ausführungsbeispielen.

Es zeigen:
- Fig. 1: ¹H-¹H COSY-Spektrum eines wässrigen Extraktes eines Ausführungsbeispiels einer erfindungsgemäßen Zusammensetzung aus einem thermoplastischen Polymer und einem erfindungsgemäßen polymeren oder oligomeren Wirkstoff.
- Fig. 2: ¹H-¹H COSY-Spektrum eines wässrigen Extraktes eines weiteren Ausführungsbeispiels einer erfindungsgemäßen Zusammensetzung aus einem thermoplastischen Polymer und einem erfindungsgemäßen polymeren oder oligomeren Wirkstoff.

### Beispiel 1 - Synthese von Poly(4,4'-methylenbis(cyclohexylamin)-guanidinhydrochlorid) (PMBCG)

In einen dreifach ausgeheizten 100 ml-Dreihalskolben wird im Argon-Gegenstrom 1 Äquivalent (8,12 g, 85 mmol) Guanidinhydrochlorid gegeben. Anschließend wird in der Glovebox 1 Äquivalent (17,88 g, 85 mmol) 4,4'-Methylenbis(cyclohexylamin)zugefügt.

Der Kolben wird mit einem Innenthermometer und einem dreifach ausgeheizten Rückflusskühler mit Rückschlagventil nach Stutz (im Folgenden Stutzkühler) ausgestattet.

In einem Ölbad wird das Reaktionsgemisch erwärmt, wobei ab einer Temperatur von 100°C eine langsame Gasentwicklung einsetzt. Bei weiterer Temperaturerhöhung wird die Gasentwicklung nur langsam stärker. Nach insgesamt 85 Minuten wird eine Temperatur von 170°C erreicht.

Diese Temperatur wird für neun Stunden beibehalten, bis die Gasentwicklung nach Augenschein beendet ist.

Unter Eiskühlung und Ölpumpenvakuum wird die Schmelze auf Raumtemperatur abgekühlt.

Die eingesetzten Ausgangsmengen liefern unter den zuvor genannten Bedingungen 24,48 g eines transparenten, farblosen und spröden Feststoffs.

Die Struktur des erhaltenen Polymers lässt sich entsprechend Formel (I) darstellen.

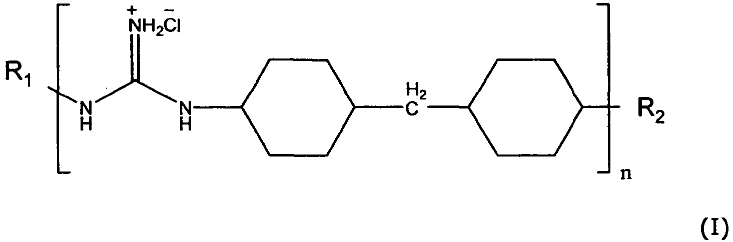

Dabei ist n = 1 bis 8, vorwiegend 1 bis 3.

Die Reste R1 und R2 können sowohl von dem eingesetzten Monomer als auch von dem eingesetzten Guanidinhydrochlorid stammen und sind daher wie folgt definiert:
R1 ist ausgewählt aus H oder 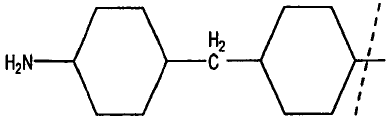 und
R2 ist ausgewählt aus NH₂ oder

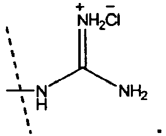

Das entstehende Produktgemisch enthält somit polymere Verbindungen entsprechend den Formeln (II), (III) und (IV): 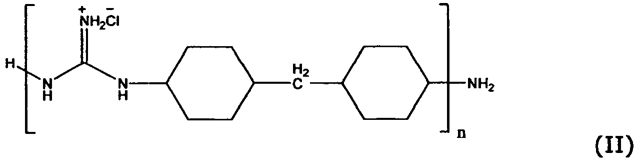 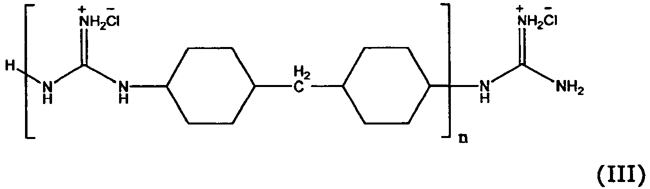 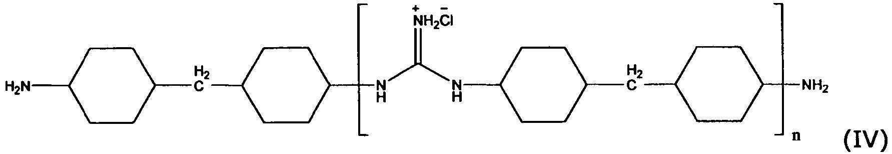 wobei n definiert ist wie in Formel (I).

### Beispiel 2 - Synthese eines Homopolymers auf Basis von Diethylentriamin

In einem dreifach ausgeheizten und mit Argon befüllten 100 ml-Dreihalskolben mit Innenthermometer, Stutzkühler und einem Absaugstück mit Hahn werden 1 Äquivalent (8,12 g, 85 mmol) Guanidinhydrochlorid und 1 Äquivalent (8,77 g, 85 mmol) Diethylentriamin mit Hilfe eines Ölbades innerhalb von 50 Minuten auf eine Temperatur von 150°C erwärmt.

Ab dem Erreichen einer Temperatur von 95°C ist eine Gasentwicklung zu beobachten, die bei weiterer Erhöhung der Temperatur schnell zunimmt.

Die Schmelze wird unter Rühren für fünf Stunden bei 150°C gehalten bis die Gasentwicklung beendet ist.

Unter Eiskühlung und Ölvakuum wird die Schmelze auf Raumtemperatur abgekühlt.

Die eingesetzten Ausgangsmengen liefern unter den zuvor genannten Bedingungen 11,96 g eines weißen und spröden Feststoffs.

Überraschenderweise zeigt die repetitive Monomereinheit des entstehenden polymeren Wirkstoffs die zyklische Struktur entsprechend Formel (V):

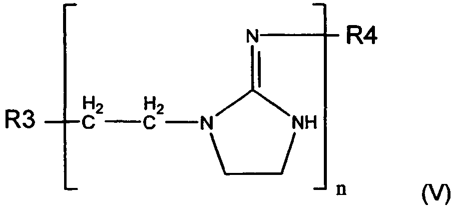

Dabei ist n = 1 bis 12, vorwiegend 2 bis 8. R3 ist entweder NH₂ oder 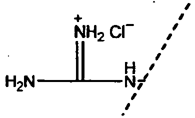 und R4 ist ausgewählt aus 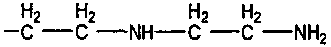 oder

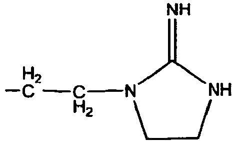

Das entstehende Produktgemisch enthält somit polymere Verbindungen entsprechend den Formeln (VI), (VII) und (VIII):

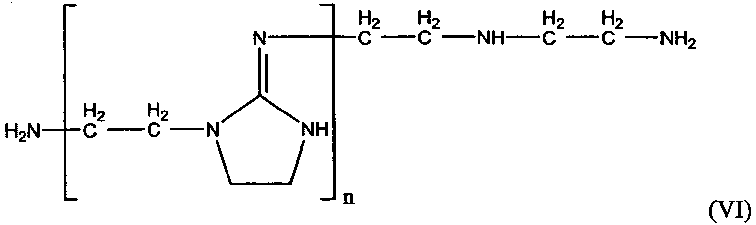

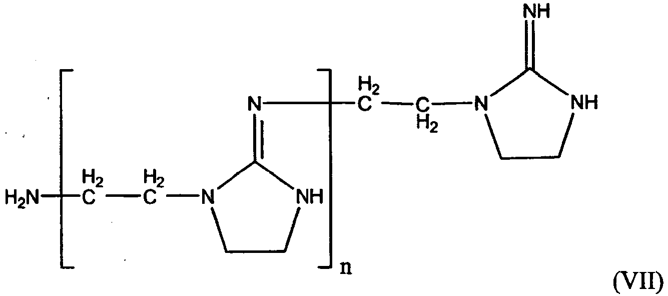

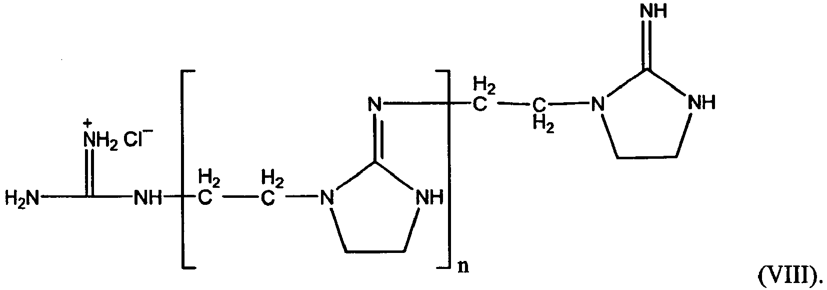

Dabei ist vorstellbar, dass etwa 90 % der Ringe in den Formeln (VI), (VII) und (VIII) eine positive Ladung tragen. Denkbar ist dabei auch, dass die positive

Ladung nicht auf einem der Stickstoffatome im Ring lokalisiert sondern vielmehr delokalisiert ist. Eine alternative Darstellungsform der Formel (VIII) ist mithin die folgende Formel (VIII')

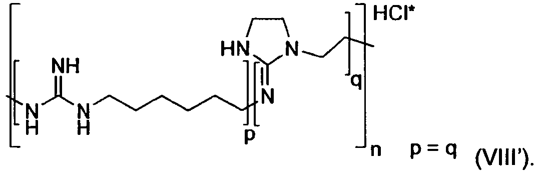

Auch die Formeln (I) bis (IV) des Beispiels 2 lassen sich in analoger Weise darstellen, wobei die positive Ladung mesomer auf alle drei Stickstoffatome der Guanidineinheit verteilt ist.

### Beispiel 3 - Synthese von Guanidin-Copolymeren

In einem entsprechend den vorbeschriebenen Beispielen vorbereiteten Reaktionskolben werden jeweils 1 Äquivalent (8,12 g, 85 mmol) Guanidinhydrochlorid und 1 Äquivalent der Comonomere, die in einem Mischungsverhältnis entsprechend Tabelle 1 vorliegen, gemeinsam mit Hilfe eines Ölbades innerhalb von 30 Minuten auf eine Temperatur von 170°C erwärmt.

Die Schmelze wird unter Rühren für fünf Stunden bei dieser Temperatur gehalten. Unter Eiskühlung und Ölvakuum wird die Schmelze auf Raumtemperatur abgekühlt.

**Tabelle 1: Mischungsverhältnisse der in dem Amin-Gemisch eingesetzten Di- und Triamine zur Herstellung von Guanidin-Copolymeren (Äq = Äquivalent).**

| Nr. | Monomer 1 | Monomer 2 | Eingesetzte Menge Monomer 1 | Eingesetzte Menge Monomer 2 |
|---|---|---|---|---|
| C1 | 4,4'-Methylenbis(cyclohexyl-amin) | Diethylentriamin | 14,30 g | 2,21 g |
| | | | 68 mmol | 17 mmol |
| | | | 0,80 Äq | 0,2 Äq |
| C2 | 4,4'-Methylenbis(cyclohexylamin) | Diethylentriamin | 13,41 g | 2,77 g |
| | | | 63,75 mmol | 21,25 mmol |
| | | | 0,75 Äq | 0,25 Äq |
| C3 | 4,4'-Methylenbis(cyclohexylamin) | Diethylentriamin | 11,92 g | 3,69 g |
| | | | 56,67 mmol | 28,33 mmol |
| | | | 0,67 Äq | 0,33 Äq |
| C4 | 4,4'-Methylenbis(cyclohexylamin) | Diethylentriamin | 8,94 g | 5,53 g |
| | | | 42,50 mmol | 42,50 mmol |
| | | | 0,50 Äq | 0,50 Äq |
| C5 | 4,4'-Methylenbis(cyclohexylamin) | Hexamethylendiamin | 3,58 g | 7,90 g |
| | | | 17,00 mmol | 68,00 mmol |
| | | | 0,20 Äq | 0,80 Äq |
| C6 | 4,4'-Methylenbis(cyclohexylamin) | Hexamethylendiamin | 4,47 g | 7,41 g |
| | | | 21,25 mmol | 63,75 mmol |
| | | | 0,25 Äq | 0,75 Äq |
| C7 | 4,4'-Methylenbis(cyclohexylamin) | Hexamethylendiamin | 5,96 g | 6,59 g |
| | | | 28,33 mmol | 56,67 mmol |
| | | | 0,33 Äq | 0,67 Äq |
| C8 | 4,4'-Methylenbis(cyclohexylamin) | Hexamethylendiamin | 8,94 g | 4,94 g |
| | | | 42,50 mmol | 42,50 mmol |
| | | | 0,50 Äq | 0,50 Äq |
| C9 | 4,4'-Methylenbis(cyclohexylamin) | Hexamethylendiamin | 11,92 g | 3,29 g |
| | | | 56,67 mmol | 28,33 mmol |
| | | | 0,67 Äq | 0,33 Äq |
| C10 | 4,4'-Methylenbis(cyclohexylam in) | Hexamethylendiamin | 13,41 g | 2,47 g |
| | | | 63,75 mmol | 21,25 mmol |
| | | | 0,75 Äq | 0,25 Äq |
| C11 | 4,4'-Methylenbis(cyclohexylamin) | Hexamethylendiamin | 14,30 g | 1,98 g |
| | | | 68,00 mmol | 17,00 mmol |
| | | | 0,80 Äq | 0,20 Äq |
| C12 | 4,4'-Methylenbis(cyclohexylam in) | Triethylenglycoldiamin | 13,41 g | 3,15 g |
| | | | 63,75 mmol | 21,25 mmol |
| | | | 0,75 Äq | 0,25 Äq |
| C13 | 4,4'-Methylenbis(cyclohexylamin) | Triethylenglycoldiamin | 11,92 g | 4,20 g, |
| | | | 56,67 mmol | 28,33 mmol |
| | | | 0,67 Äq | 0,33 Äq |
| C14 | 4,4'-Methylenbis(cyclohexylamin) | Triethylenglycoldiamin | 8,94 g | 6,30 g |
| | | | 42,50 mmol | 42,50 mmol |
| | | | 0,50 Äq | 0,50 Äq |
| C15 | 4,4'-Methylenbis(cyclohexylam in) | Triethylenglycoldiamin | 5,96 g | 8,40 g |
| | | | 28,33 mmol | 56,67 mmol |
| | | | 0,33 Äq | 0,67 Äq |
| C16 | 4,4'-Methylenbis(cyclohexylamin) | Triethylenglycoldiamin | 4,47 g | 9,45 g |
| | | | 21,25 mmol | 63,75 mmol |
| | | | 0,25 Äq | 0,75 Äq |
| C17 | Diethylentriamin | Hexamethylendiamin | 1,75 g | 7,90 g |
| | | | 17,00 mmol | 68,00 mmol |
| | | | 0,20 Äq | 0,80 Äq |
| C18 | Diethylentriamin | Hexamethylendiamin | 2,19 g | 7,41 g |
| | | | 21,25 mmol | 63,75 mmol |
| | | | 0,25 Äq | 0,75 Äq |
| C19 | Diethylentriamin | Hexamethylendiamin | 3,69 g | 6,59 g |
| | | | 28,33 mmol | 56,67 mmol |
| | | | 0,33 Äq | 0,67 Äq |
| C20 | Diethylentriamin | Hexamethylendiamin | 5,53 g | 4,94 g |
| | | | 42,50 mmol | 42,50 mmol |
| | | | 0,50 Äq | 0,50 Äq |
| C21 | Diethylentriamin | Triethylenglycoldi amin | 8,30 g | 3,15 g |
| | | | 63,75 mmol | 21,35 mmol |
| | | | 0,75 Äq | 0,25 Äq |
| C22 | Diethylentriamin | Triethylenglycoldiamin | 7,38 g | 4,20 g |
| | | | 56,67 mmol | 28,33 mmol |
| | | | 0,67 Äq | 0,33 Äq |
| C23 | Diethylentriamin | Triethylenglycoldi amin | 5,53 g | 6,30 g |
| | | | 42,50 mmol | 42,50 mmol |
| | | | 0,50 Äq | 0,50 Äq |

### Beispiel 4 - Bestimmung der minimalen Hemmkonzentration der erfindungsgemäß verwendeten polymeren Guanidinderivate.

Zur Überprüfung der bioziden Wirkung der erfindungsgemäß verwendeten polymeren Guanidinderivate werden die Verbindungen, die entsprechend einem der vorangegangenen Beispiele hergestellt werden in einem Bakterien-Nährmedium, vorzugsweise Tryptic Soy Broth, angesetzt und auf verschiedene Konzentrationen verdünnt.

Diese Lösungen unterschiedlicher Konzentration werden mit einer Suspension von *Escherichia coli* inokuliert und für 24 h bei 37°C inkubiert.

Unter der minimalen Hemmkonzentration (MHK) wird dann die geringste Konzentration des zu untersuchenden Biozids in der Lösung verstanden, bei der das Wachstum der Bakterien gehemmt wird. Bei der entsprechenden Lösung ist dann keine Eintrübung durch das Wachstum der Bakterien beobachtbar.

Für die in Beispiel 1 und Beispiel 2 dargestellten Homopolymere entsprechend Formel (I) und Formel (V) sowie für die aus den in Beispiel 3 genannten Comonomer-Mischungen C1 bis C23 erhaltenen Copolymere werden die in Tabelle 2 aufgeführten mittleren minimalen Hemmkonzentrationen (MHK) erhalten.

**Tabelle 2: Bestimmung der minimalen Hemmkonzentration erfindungsgemäß verwendeter polymerer Guanidinderivate (MHK = minimale Hemmkonzentration).**

| **Verbindung** | **MHK [µg/ml]** | **Verbindung** | **MHK [µg/ml]** |
|---|---|---|---|
| Kontrollpolymer | 5 | C11 | 9,75 |
| Entspr. Formel (I) | 5 | C12 | 5,5 |
| Entspr. Formel (V) | >250 | C13 | 8,5 |
| C1 | 7,5 | C14 | 10 |
| C2 | 22,5 | C15 | 10 |
| C3 | 25 | C16 | 10 |
| C4 | 50 | C17 | 3 |
| C5 | 1,5 | C18 | 10 |
| C6 | 4,7 | C19 | 10 |
| C7 | 4,25 | C20 | 40 |
| C8 | 2,5 | C21 | >50 |
| C9 | 3,5 | C22 | >50 |
| C10 | 2,5 | C23 | >50 |

Als Kontrolle wurde ein Kontrollpolymer eingesetzt, dessen biozide Wirkung bekannt ist und dessen minimale Hemmkonzentration üblicherweise bei 5 µg/ml liegt.

Man erkennt, dass alle erfindungsgemäß verwendeten polymeren Guanidinderivate, insbesondere die erfindungsgemäß verwendeten Copolymere eine biozide Wirkung aufweisen. Dabei weisen insbesondere Copolymere, die als zweites Monomer Hexametyhlendiamin aufweisen, eine minimale Hemmkonzentration auf, die sogar unterhalb von 5 µg/ml liegt:

**Tabelle 3: Ausgewählte erfindungsgemäß verwendete Copolymere mit besonders niedriger minimaler Hemmkonzentration (MHK). (MBC = 4,4'-Methylenbis(cyclohexylamin), HMD = Hexamethylendiamin, DETA = Diethylentriamin).**

| **Copolymer** | **Monomer 1** | **Monomer 2** | **Mischungsverhältnis** | **Reaktionsbedingungen** | **MHK** |
|---|---|---|---|---|---|
| C5 | MBC | HMD | 1:4 | 5h, 170°C | 1,5 |
| C6 | MBC | HMD | 1:3 | 5h, 170°C | 4,7 |
| C7 | MBC | HMD | 1:2 | 5h, 170°C | 4,25 |
| C8 | MBC | HMD | 1:1 | 5h, 170°C | 2,5 |
| C9 | MBC | HMD | 2:1 | 5h, 170°C | 3,5 |
| C10 | MBC | HMD | 3:1 | 5h, 170°C | 2,5 |
| C17 | DETA | HMD | 1:4 | 5h, 170°C | 3 |

Man erkennt, dass es bei den erfindungsgemäß verwendeten polymeren oder oligomeren Wirkstoffen mit biozider Wirkung, wobei der Wirkstoff das Produkt einer Polykondensation eines Guanidin-Säureadditionssalzes mit einem Amin-Gemisch, welches wenigstens ein Diamin und/oder ein Triamin enthält, besonders günstig ist, wenn wenigstens ein Amin ausgewählt ist aus 4,4'-Methylenbis(cyclohexylamin) und Diethylentriamin. Zweckmäßig ist dabei das Guanidin-Säureadditionssalz Guanidinhydrochlorid.

Man erkennt weiter, dass das polymere oder oligomeren Wirkstoff ein Homopolymer sein kann. Dabei ist es günstig, wenn das Amin-Gemisch aus dem Triamin Diethylentriamin besteht oder wenn das Amin-Gemisch aus dem Diamin 4,4'-Methylenbis(cyclohexylamin) besteht.

Man erkennt auch, dass das Amin-Gemisch eine erste Komponente und wenigstens eine zweite Komponente enthalten kann, wobei die erste Komponente ein Diamin oder ein Triamin ist, ausgewählt aus der Gruppe 4,4'-Methylenbis(cylcohexylamin), Diethylentriamin, und wobei die zweite Komponente ein Diamin oder ein Triamin, ausgewählt aus der Gruppe 4,4'-Methylenbis(cylcohexylamin), Diethylentriamin, Hexamethylen-diamin, Triethylenglycol-diamin, ist und wobei die erste Komponente von der zweiten Komponente verschieden ist.

Besonders bevorzugt ist die erste Komponente 4,4'-Methylenbis(cylcohexylamin) und die zweite Komponente ist ausgewählt aus Diethylentriamin, Hexamethylendiamin, Triethylenglycoldiamin. Günstig ist es auch, wenn die erste Komponente Diethylentriamin ist und die zweite Komponente ausgewählt ist aus Hexamethylendiamin und Triethylenglycoldiamin.

Dabei liegen die erste Komponente und die zweite Komponente bevorzugt in einem Mischungsverhältnis von 4:1 bis 1:4 vor. Das Amin-Gemisch und das Guanidin-Salz werden bevorzugt in etwa äquimolar zueinander eingesetzt.

Man erkennt, dass es bei den erfindungsgemäß verwendeten polymeren oder oligomeren Wirkstoffen, wobei der Wirkstoff das Produkt einer Polykondensation eines Guanidin-Säureadditionssalzes mit einem Amin-Gemisch, welches wenigstens ein Diamin und/oder ein Triamin enthält, besonders vorteilhaft ist, wenn sie nach einem Verfahren hergestellt sind, umfassend die Schritte Vorlegen von etwa einem Äquivalent Guanidinhydrochlorid, Zugeben etwa eines Äquivalents einer Amin-Mischung, welche eine oder zwei der Verbindungen der Gruppe umfassend Diamin, das wenigstens einen cycloaliphatischen Rest aufweist, und Dialkylentriamin, enthält, Erhitzen auf 150 bis 170°C und Rühren der Schmelze bei 150 bis 170°C bis die Gasentwicklung beendet ist, wenigstens aber für 5 Stunden.

### Beispiel 5 - Verknüpfungsprodukt mit kovalent an ein thermoplastisches Polymer (Komponente b)) gebundenem polymerem oder oligomerem Wirkstoff (Komponent a))

Bei der Eincompoundierung polymerer oder oligomerer Wirkstoffe in thermoplastische Polymere wie z.B. Polyurethane kommt es unter bestimmten Bedingungen zu einem sogenannten Reactive processing. Dabei werden die polymeren oder oligomeren Wirkstoffe kovalent mit dem verwendenten Thermoplastenverknüpft.

In einem entsprechenden Herstellungsverfahren werden dazu wässrige Lösungen der polymeren oder oligomeren Wirkstoffe mit Schmelzen thermoplastischer Polykondensate oder Polyadditionsprodukte coextrudiert. Dabei entstehen durch Umkondensation neue Thermoplaste (biozide Verknüpfungsprodukte).

Als Ausgangs-Thermoplaste werden Polykondensate oder Polyadditionsprodukte eingesetzt, die durch Schmelzextrusion verarbeitbar sind, z. B. Polyester wie Polyethylenterephthalt, Polybutylenterephthalat, Polylaktid, Polycaprolakton; Polyamide wie z. b. Polyamid(PA) 6, PA 66, PA 610, PA11, PA12; Polyesteramide, aliphatische und aromatische Polycarbonate, aliphatische und aromatische Polyurethane und Polyharnstoffe.

Als polymere bzw. oligomere Wirkstoffe werden wasserlösliche Polyguanidine (PG) eingesetzt, bei denen es sich insbesondere um solche mit folgenden Strukturen handelt: 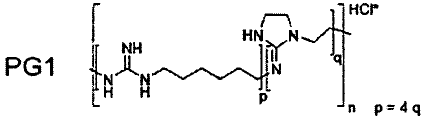 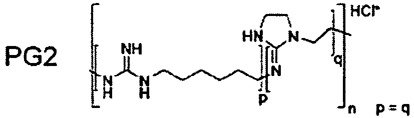 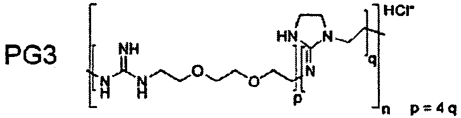 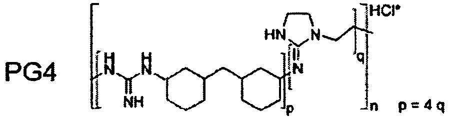 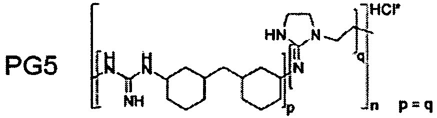 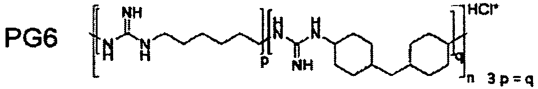 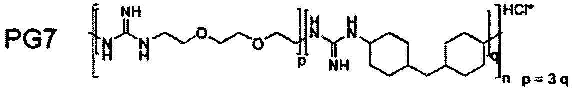 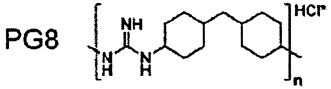 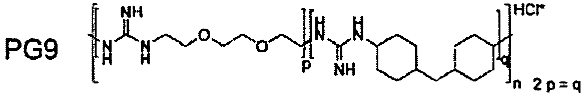 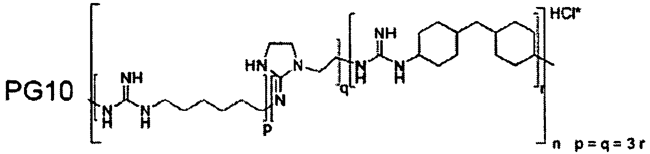 wobei
HCl* bedeutet, dass das HCl nicht kovalent gebunden ist,
n ist eine natürliche Zahl, vorzugsweise von 1 bis 20, weiter bevorzugt von 2 bis 16 und insbesondere von 3 bis 8,
p, q und r sind ganze Zahlen, die das bevorzugte molare Verhältnis der Strukturfragmente zueinander in den Formeln definieren.

Zur Herstellung der bioziden Verknüpfungsprodukte wird der Thermoplast in Schmelzextrusion in einem herkömmlichen Extruder bei Extrusionstemperaturen von 70 - 300°C je nach Thermoplast mit einer wässrigen Lösung des bioziden polymeren oder oligomeren Wirkstoffs mit Konzentrationen von 0,1 - 90 Gew. % des bioziden polymeren oder oligomeren Wirkstoffs in Wasser additiviert.

Das Wasser wird in der Verdampfungszone des Extruders entfernt. Als neues Produkt wird am Ende der Extrusion das biozide Verknüpfungsprodukt erhalten, der 0,1 - 50 % der Strukturen der polymeren oder oligomeren Wirkstoffe relativ zum eingesetzten Thermoplasten enthält. Die polymeren oder oligomeren Wirkstoffe sind dabei durch Umkondensation kovalent an den Thermoplasten gebunden. Die Umkondensation findet während der Extrusion statt.

Der Nachweis, dass die polymeren oder oligomeren Wirkstoffe nach der Extrusion kovalent an das eingesetzte thermoplastische Polymer gebunden sind, kann sowohl mittels NMR als auch mittels Massenspektrometrie gezeigt werden.

In einem ersten Ausführungsbeispiel für die Verknüpfungsprodukte mit kovalent an ein thermoplastisches Polymer gebundenem polymerem oder oligomerem Wirkstoff wird thermoplastisches aliphatisches Polyurethan (TAPU) bei einer Massetemperatur von 170°C im Zweischneckenextruder extrudiert und mit einer 40%igen wässrigen Lösung eines ersten Polyguanidins (PG1) additiviert, welches folgende Struktur aufweist:

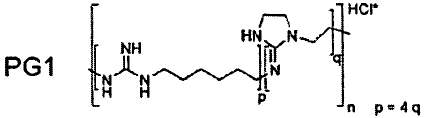

Die Additivierung erfolgt dabei so, dass ein Compound mit 10 Gew. % des ersten Polyguanidins (PG1) in TAPU entsteht.

Die massenspektroskopische Analyse zeigt, dass nach der Extraktion des Compounds kein Wirkstoff, nämlich kein erstes Polyguanidin (PG1) im Exktrakt zu finden ist. Dabei wurde die Extraktion mit siedendem Wasser durchgeführt.

In einem zweiten Ausführungsbeispiel wird als thermoplastisches Polymer anstelle des thermoplastischen aliphatischen Polyurethans (TAPU) ein thermoplastisches aliphatisches/aromatische Polyurethan (TAAPU) verwendet. Die Extrusion erfolgte auch hier bei einer Massetemperatur von 170°C im Zweischneckenextruder unter Additivierung einer 40%igen wässrigen Lösung des ersten Polyguanidins (PG1).

Auch hier kann kein reines PG1 im Extrakt nachgewiesen werden, was die kovalente Bindung von PG1 an das verwendete thermoplastische aliphatische/aromatische Polyurethan zeigt.

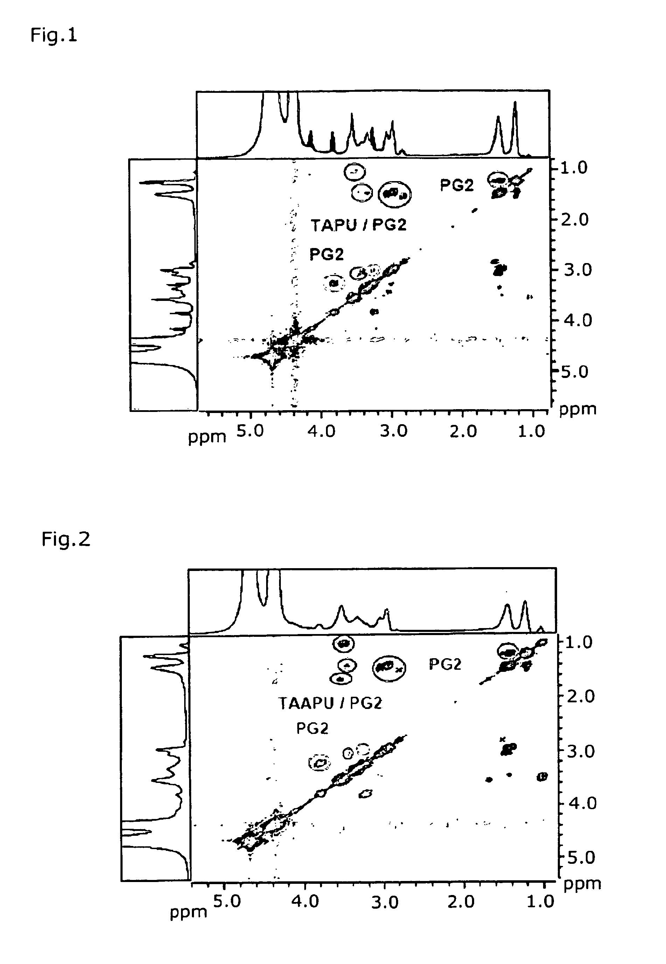

In Fig. 1 ist auch für ein drittes Ausführungsbeispiel die kovalente Anbindung eindeutig im zweidimensionalen NMR-Spektrum erkennbar. Bei diesem Beispiel wurde thermoplastisches aliphatisches Polyurethan (TAPU) wie zuvor bei einer Massetemperatur von 170°C im Zweischneckenextruder extrudiert und mit einer 40%igen wässrigen Lösung eines zweiten Polyguanidins (PG2) additiviert. Das zweite Polyguanidin (PG2) weist dabei folgende Struktur auf:

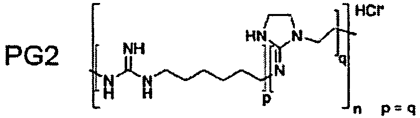

Es entsteht ein Compound mit 10 Gew.-% des zweiten Polyguanidins (PG2) in TAPU. Fig. 1 zeigt dabei das ¹H-¹H COSY-Spektrum des Exktraktes des Ausführungsbeispiels.

Für ein viertes Ausführungsbeispiel zeigt auch Fig. 2 das ¹H-¹H COSY Spektrum des Extraktes. Dabei wurde anstelle des thermoplastischen aliphatischen Polyurethans (TAPU) ein thermoplastisches aliphatisches/aromatisches Polyurethan verwendet und wie zuvor mit einer 40%igen wässrigen Lösung des zweiten Polyguanidins (PG2) bei einer Massetemperatur von 170°C extrudiert. Die Additivierung erfolgte derart, dass ein Compound mit 10 Gew-% des zweiten Polyguanidins (PG2) in TAAPU entsteht. Auch hier ist kein polymerer oder oligomerer Wirkstoff, nämlich kein zweites Polyguanidin (PG2), im Extrakt mit siedendem Wasser nachweisbar.

### Versuchbedingungen für 1H-1H-COSY NMR:

1H-1H-COSY NMR wurden am Bruker Avance 300 B Spektrometer gemessen (Dual 1H - 13C Probenkopf mit Z-Gradient), je bei 25 °C in einem Lösungsmittelgemisch aus Hexafluoroisopropanol (ca. 1 mL) und D₂O (ca. 0,2 mL). Kalibriert wurde auf den protonierten D₂O-Peak als internen Standard (4.79 ppm bei 25 °C). (Die in dem Bericht dargestellten 2D-Spektren sind nicht kalibriert, weil sich die 1D-Spektren oben und links dadurch verschieben).

### Versuchsbedingungen Massenspektroskopie:

Die Massenspektren wurden durch chemische Ionisation bei Atmosphärendruck (atmospheric pressure chemical ionization, APCI) gemessen an einem Thermo Fisher Scientific Finnigan LTQ-FT Spektrometer in Methanol als Lösungsmittel.

Die Erfindung ist nicht auf eine der vorbeschriebenen Ausführungsformen beschränkt, sondern in vielfältiger Weise abwandelbar.

### Beispiel 6 - Allgemeine Vorschrift zur Fertigung eines Katheterschlauchs auf Polyurethan-Basis aus mit dem erfindungsgemäß einzusetzenden polymeren oder oligomeren Wirkstoff ausgestatteten TPU Granulat

Es wird ein aliphatisches thermoplastisches Polyurethan (auf Basis eines Polytetramethylen-Glycolethers) mit 10 bis 35 Gew.-%, bezogen auf die Gesamtmischung, Bariumsulfat mit einer mittleren Teilchengröße von 0,01 µm bis 10 µm sowie 0,5 bis 10 Gew.-%, bezogen auf den Anteil der Komponente a), eines erfindungsgemäß zu verwendenden Verknüpfungsprodukt gemischt und anschließend extrudiert. Die Extrusion erfolgt mit für die Katheterherstellung üblichen Extrudern, beispielsweise dem Extruder Maillefer Typ ED 45-30D.

## Patentansprüche

1. Verwendung eines bioziden Verknüpfungsprodukts aus
a) einen polymeren oder oligomeren Wirkstoff mit biozider Wirkung, welcher erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht, und
b) einem thermoplastischen Polymer
für die Herstellung medizinischer Artikel.

2. Verwendung eines bioziden Verknüpfungsprodukts, welches erhältlich ist durch Reaktion von
a) einem polymeren oder oligomeren Wirkstoff mit biozider Wirkung, welcher erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht,
mit
b) einem thermoplastischen Polymer,
für die Herstellung medizinischer Artikel.

3. Verwendung mindestens einem der Ansprüche 1 oder 2, **dadurch gekennzeichnet, dass** das Amingemisch ein Alkylendiamin, insbesondere eine Verbindung der allgemeinen Formel
NH₂(CH₂)ₙNH₂
in welcher n eine ganze Zahl zwischen 2 und 10, insbesondere 6, ist, umfasst.

4. Verwendung gemäß mindestens einem der Ansprüche 1 bis 3, **dadurch gekennzeichnet, dass** das Amingemisch eine erste Komponente und wenigstens eine zweite Komponente enthält, wobei
a) die erste Komponente ein Diamin oder ein Triamin ist, das ausgewählt ist aus der Gruppe, die aus
i) Diamin, das wenigstens einen cycloaliphatischen Rest aufweist,
und
ii) Dialkylentriamin besteht, und wobei
b) die zweite Komponente ein Diamin oder ein Triamin ist, das ausgewählt ist aus der Gruppe, die aus
i) Diamin, das wenigstens einen cycloaliphatischen Rest aufweist,
ii) Dialkylentriamin,
iii) Alkylendiamin und
iv) Oxyalkylendiamin besteht, und
wobei die erste Komponente von der zweiten Komponente verschieden ist.

5. Verwendung gemäß mindestens einem der Ansprüche 1 bis 4, **dadurch gekennzeichnet, dass** der polymere oder oligomere Wirkstoff ein Iminoimidazolderivat ist.

6. Verwendung gemäß mindestens einem der Ansprüche 1 bis 5, **dadurch gekennzeichnet, dass** der polymere oder oligomere Wirkstoff eine Struktur aufweist, die aus der Gruppe umfassend 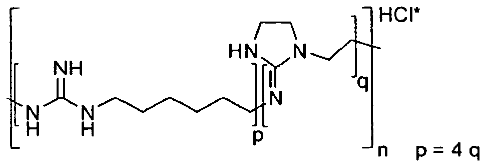 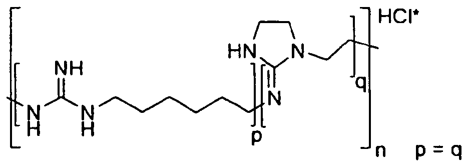 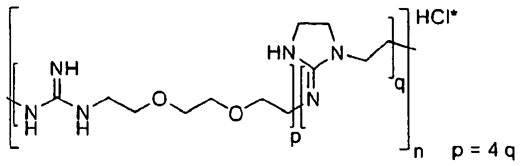 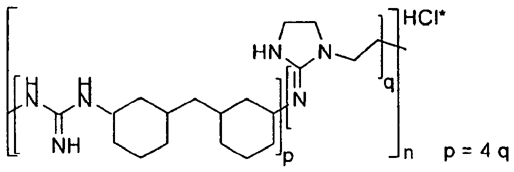 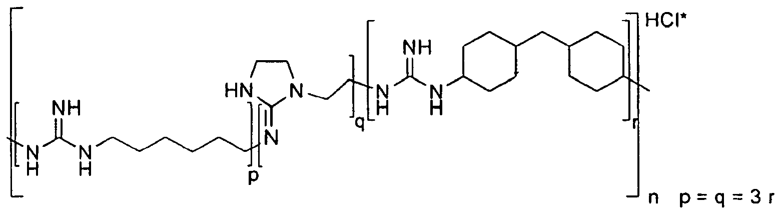 ausgewählt ist, wobei
HCl* bedeutet, dass das HCl nicht kovalent gebunden ist,
n ist eine natürliche Zahl, vorzugsweise von 1 bis 20, weiter bevorzugt von 2 bis 16 und insbesondere von 3 bis 8,
p, q und r sind ganze Zahlen, die das bevorzugte molare Verhältnis der Strukturfragmente zueinander in den Formeln definieren.

7. Verwendung gemäß mindestens einem der Ansprüche 1 bis 6, **dadurch gekennzeichnet, dass** der polymere oder oligomere Wirkstoff ein mittleres Molekulargewicht im Bereich von 500 bis 7000, insbesondere von 1000 bis 5000 aufweist.

8. Verwendung gemäß mindestens einem der Ansprüche 1 bis 7, **dadurch gekennzeichnet, dass** das thermoplastische Polymer ausgewählt ist aus der Gruppe bestehend aus Polyurethan, Polycarbonat, Polyethersulfon, Silikon, Polyester, Polyamid, Polylactid und Polyharnstoff.

9. Verwendung gemäß mindestens einem der Ansprüche 1 bis 8, **dadurch gekennzeichnet, dass** das thermoplastische Polymer ein thermoplastisches aliphatisches Polyurethan (TAPU) oder ein thermoplastisches aliphatisches/aromatisches Polyurethan (TAAPU) ist und dass der polymere oder oligomere Wirkstoff eine cyclische Struktur aufweist, die ausgewählt ist aus der Gruppe, umfassend 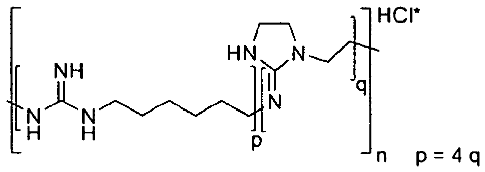 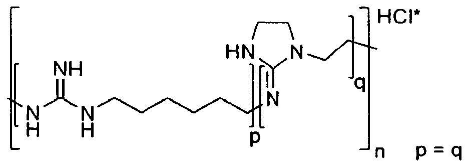 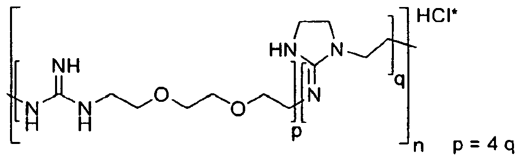 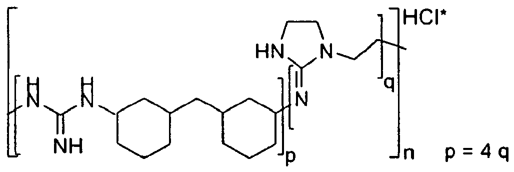 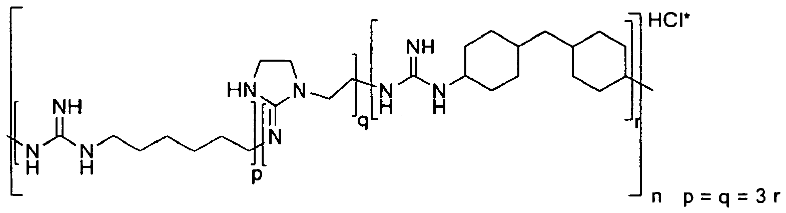 wobei
HCl* bedeutet, dass das HCl nicht kovalent gebunden ist,
n ist eine natürliche Zahl, vorzugsweise von 1 bis 20, weiter bevorzugt von 2 bis 16 und insbesondere von 3 bis 8,
p, q und r sind ganze Zahlen, die das bevorzugte molare Verhältnis der Strukturfragmente zueinander in den Formeln definieren.

10. Verwendung gemäß mindestens einem der Ansprüche 1 bis 9, **dadurch gekennzeichnet dass** der medizinische Artikel ausgewählt ist aus der Gruppe bestehend aus zentralvenösen Kathetern; peripheren Venenkathetern; Beatmungsschläuche; Stents; Produkten für Anwendung in der Regionalanästhesie, insbesondere Katheter, Kuppelungen, Filter; Produkten für die Infusionstherapie, insbesondere Behältnisse, Ports, Überleitungssysteme, Filter; Zubehör wie Konnektoren, Spikes, Ventile, Dreiwegehähne, Spritzen, Leitungen, Zuspritzports; Produkte der Zubereitung, insbesondere Transfersets, Mixsets; urologische Produkte, insbesondere Katheter, Urinmess- und -sammelgeräte; Wunddrainage; Wundversorgung; chirurgische Nahtmaterialien; Implantatzubehörteile und Implantate, insbesondere Kunstoffimplantate, beispielsweise Herniennetze, Vliese, Gestricke, Gewirke, Ports, Portkatheter, Gefäßprothesen; Desinfektionsmittel; chirurgisches Einmalinstrumentarium; Thoraxdrainagen; Sonden; Katheter; Gehäuse von medizinischen Geräten, insbesondere Infusionspumpen, Dialysegeräte und Monitore, künstliche Gebisse; Behälter für Flüssigkeiten, insbesondere Kontaktlinsenbehälter; Reinigungsmittel und Desinfektionsmittel.

11. Verfahren zur Herstellung eines medizinischen Artikels, umfassend die folgenden Schritte
a) Zusammenführen und Vermischen des bioziden Verknüpfungsprodukts, wie in mindestens einem der Ansprüche 1 bis 9 definiert, mit gegebenenfalls einem thermoplastischen Polymer und
b) Einsetzen des unter a) erhaltenen Gemisches in einem oder mehreren Formgebungsverfahren unter Ausbildung eines medizinischen Artikels.

12. Verfahren gemäß Anspruch 11, **dadurch gekennzeichnet, dass** das biozide Verknüpfungsprodukt dem Formgebungsverfahren in Schritt b) als Granulat oder Masterbatch zugeführt wird.

13. Verfahren gemäß Anspruch 11 oder 12, **dadurch gekennzeichnet, dass** das Formgebungsverfahren eine Extrusion ist.

14. Medizinischer Artikel umfassend ein biozides Verknüpfungsprodukt wie in mindestens einem der Ansprüche 1 bis 9 definiert.

15. Medizinischer Artikel gemäß Anspruch 14, **dadurch gekennzeichnet, dass** es ein schlauchförmiger medizinischer Artikel, insbesondere ein Katheter, ist.

## Claims

1. Use of a biocidal linkage product of
a) a polymeric or oligomeric active ingredient with biocidal activity that is obtainable by the polycondensation of a guanidine acid addition salt with a mixture of amines containing at least one diamine and/or triamine, wherein at least one amine is selected from the group consisting of
i) a diamine having at least one cycloaliphatic residue; and
ii) dialkylene triamine, and
b) a thermoplastic polymer;
for preparing medical articles.

2. The use of a biocidal linkage product obtainable by the reaction of
a) a polymeric or oligomeric active ingredient with biocidal activity that is obtainable by the polycondensation of a guanidine acid addition salt with a mixture of amines containing at least one diamine and/or triamine, wherein at least one amine is selected from the group consisting of
i) a diamine having at least one cycloaliphatic residue; and
ii) dialkylene triamine, with
b) a thermoplastic polymer;
for preparing medical articles.

3. The use according to at least one of claims 1 or 2, **characterized in that** said mixture of amines comprises an alkylene diamine, especially a compound of general formula
NH₂(CH₂)ₙNH₂
in which n represents an integer of from 2 to 10, especially 6.

4. The use according to at least one of claims 1 to 3, **characterized in that** said mixture of amines contains a first component and at least one second component, wherein
a) the first component is a diamine or triamine selected from the group consisting of
i) a diamine having at least one cycloaliphatic residue; and
ii) dialkylene triamine, and wherein
b) the second component is a diamine or triamine selected from the group consisting of
i) a diamine having at least one cycloaliphatic residue;
ii) dialkylene triamine;
iii) alkylene diamine; and
iv) oxyalkylene diamine; and
wherein the first component is different from the second component.

5. The use according to at least one of claims 1 to 4, **characterized in that** said polymeric or oligomeric active ingredient is an iminoimidazole derivative.

6. The use according to at least one of claims 1 to 5, **characterized in that** said polymeric or oligomeric active ingredient has a structure selected from the group comprising 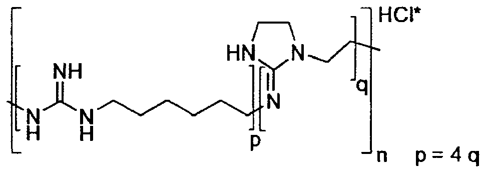 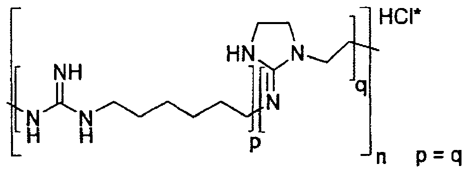 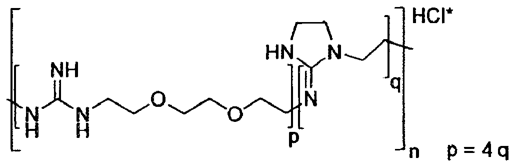 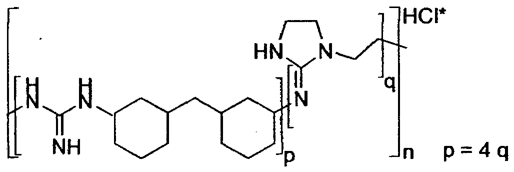 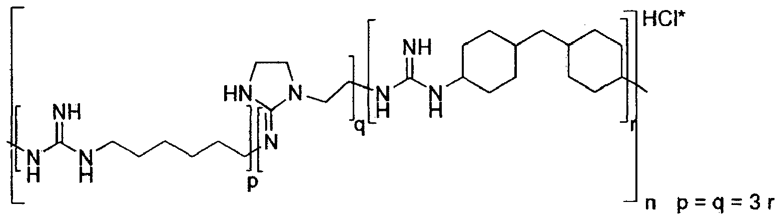 wherein
HCl* means that the HCl is not covalently bonded,
n is a natural number, preferably from 1 to 20, more preferably from 2 to 16, especially from 3 to 8,
p, q and r are integers defining the preferred molar ratio of the structural fragments in the formulas.

7. The use according to at least one of claims 1 to 6, **characterized in that** said polymeric or oligomeric active ingredient has an average molecular weight within a range of from 500 to 7000, especially from 1000 to 5000.

8. The use according to at least one of claims 1 to 7, **characterized in that** said thermoplastic polymer is selected from the group consisting of polyurethane, polycarbonate, polyethersulfone, silicone, polyester, polyamide, polylactide and polyurea.

9. The use according to at least one of claims 1 to 8, **characterized in that** said thermoplastic polymer is a thermoplastic aliphatic polyurethane (TAPU) or a thermoplastic aliphatic/aromatic polyurethane (TAAPU), and that said polymeric or oligomeric active ingredient has a cyclic structure selected from the group comprising 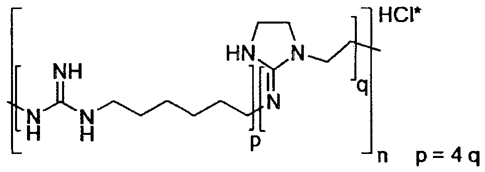 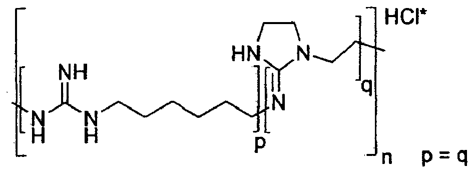 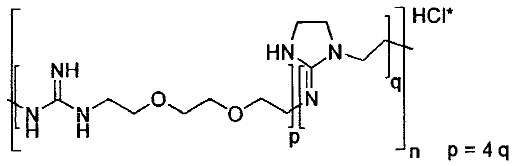 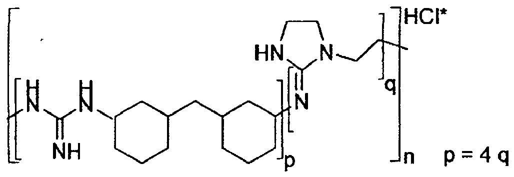 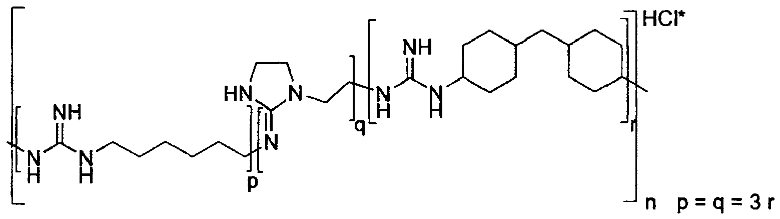 wherein
HCl* means that the HCl is not covalently bonded,
n is a natural number, preferably from 1 to 20, more preferably from 2 to 16, especially from 3 to 8,
p, q and r are integers defining the preferred molar ratio of the structural fragments in the formulas.

10. The use according to at least one of claims 1 to 9, **characterized in that** said medical article is selected from the group consisting of central venous catheters; peripheral venous catheters; breathing tubes, stents; products for application in regional anesthesia, especially catheters, couplings, filters; products for infusion therapy, especially containers, ports, conduit systems, filters; accessories, such as connectors, spikes, valves, three-way stop-cocks, syringes, conduits, injection ports; products of formulation, especially transfer sets, mixing sets; urological products, especially catheters, urine measuring and collecting devices; wound drains; wound dressing; surgical suture materials; implantation auxiliaries as well as implants, especially plastic implants, for example, hernia meshes, non-wovens, knitwear/knitted fabrics, ports, port catheters, vascular prostheses; disinfectants; disposable surgical instruments; thoracic drains; probes; catheters; housings of medical devices, especially infusion pumps, dialysis devices and screens; artificial dentures; containers for liquids, especially contact lens containers; cleaning agents and disinfectants.

11. A process for preparing a medical article, comprising the following steps:
a) combining and mixing said biocidal linkage product as defined in at least one of claims 1 to 9 with optionally a thermoplastic polymer; and
b) subjecting the mixture obtained under a) to one or more shaping methods to form a medical article.

12. The process according to claim 11, **characterized in that** said biocidal linkage product is subjected to the shaping method in step b) as pellets or as a master batch.

13. The process according to claim 11 or 12, **characterized in that** said shaping method is an extrusion.

14. A medical article comprising a biocidal linkage product as defined in at least one of claims 1 to 9.

15. The medical article according to claim 14, **characterized by** being a tubular medical article, especially a catheter.

## Revendications

1. Utilisation d'un produit de liaison biocide comprenant
a) un principe actif polymère ou oligomère à effet biocide qui peut être obtenu par la polycondensation d'un sel d'addition acide de guanidine avec un mélange d'amines contenant au moins une diamine et/ou une triamine, au moins une amine étant choisie dans le groupe consistant en
i) une diamine présentant au moins un reste cycloaliphatique,
et
ii) la dialkylène triamine, et
b) un polymère thermoplastique,
pour la production d'articles médicaux.

2. Utilisation d'un produit de liaison biocide qui peut être obtenu par la réaction de
a) un principe actif polymère ou oligomère à effet biocide qui peut être obtenu par la polycondensation d'un sel d'addition acide de guanidine avec un mélange d'amines contenant au moins une diamine et/ou une triamine, au moins une amine étant choisie dans le groupe consistant en
i) une diamine présentant au moins un reste cycloaliphatique,
et
ii) la dialkylène triamine, avec
b) un polymère thermoplastique,
pour la production d'articles médicaux.

3. Utilisation selon au moins une des revendications 1 ou 2, **caractérisée en ce que** ledit mélange d'amines comprend un alkylène diamine, notamment un composé de la formule générale
NH₂(CH₂)ₙNH₂,
où n est un nombre entier compris entre 2 et 10, notamment 6.

4. Utilisation selon au moins une des revendications 1 à 3, **caractérisée en ce que** ledit mélange d'amines contient un premier composant et au moins un second composant, dans laquelle
a) ledit premier composant est une diamine ou triamine choisie dans le groupe consistant en
i) une diamine présentant au moins un reste cycloaliphatique, et
ii) une dialkylène triamine, et dans laquelle
b) ledit second composant est une diamine ou triamine choisie dans le groupe consistant en
i) une diamine présentant au moins un reste cycloaliphatique,
ii) une dialkylène triamine,
iii) une alkylène diamine et
iv) une oxyalkylène diamine, et
dans laquelle ledit premier composant est différent dudit second composant.

5. Utilisation selon au moins une des revendications 1 à 4, **caractérisée en ce que** ledit principe actif polymère ou oligomère est un dérivé d'iminoimidazole.

6. Utilisation selon au moins une des revendications 1 à 5, **caractérisée en ce que** ledit principe actif polymère ou oligomère présente une structure choisie dans le groupe comprenant 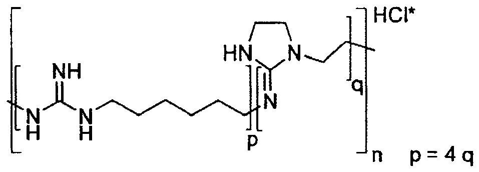 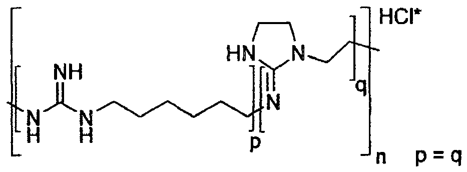 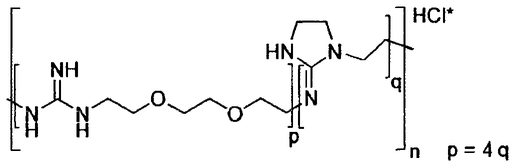 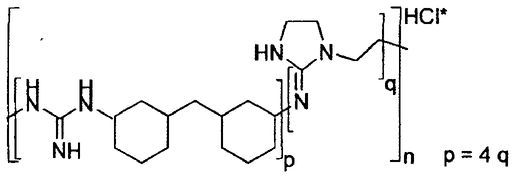 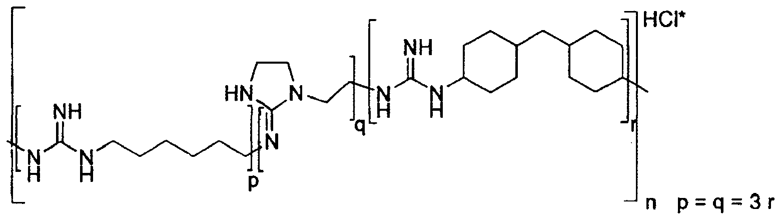 où
HCl* signifie que le HCl n'est pas lié de manière covalente,
n est un entier naturel, de préférence de 1 à 20, plus préférablement de 2 à 16, notamment de 3 à 8,
p, q et r sont des entiers définissant le rapport molaire mutuel préféré des fragments structuraux dans les formules.

7. Utilisation selon au moins une des revendications 1 à 6, **caractérisée en ce que** ledit principe actif polymère ou oligomère présente un poids moléculaire moyen compris entre 500 et 7000, notamment entre 1000 et 5000.

8. Utilisation selon au moins une des revendications 1 à 7, **caractérisée en ce que** ledit polymère thermoplastique est choisi dans le groupe consistant en polyuréthane, polycarbonate, polyéthersulfone, silicone, polyester, polyamide, polylactide et polyurée.

9. Utilisation selon au moins une des revendications 1 à 8, **caractérisée en ce que** ledit polymère thermoplastique est un polyuréthane aliphatique thermoplastique (TAPU) ou un polyuréthane aliphatique/aromatique thermoplastique (TAAPU), et **en ce que** ledit principe actif polymère ou oligomère présente une structure cyclique choisie dans le groupe comprenant 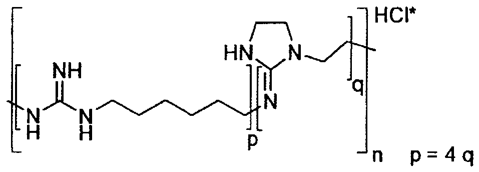 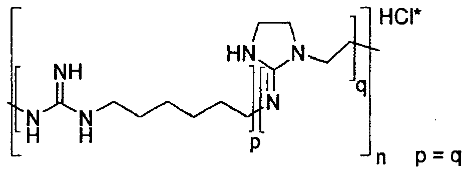 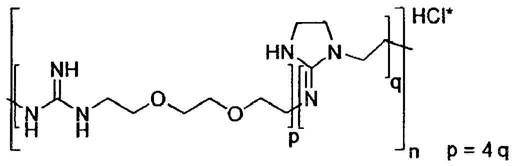 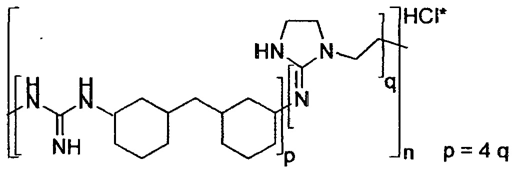 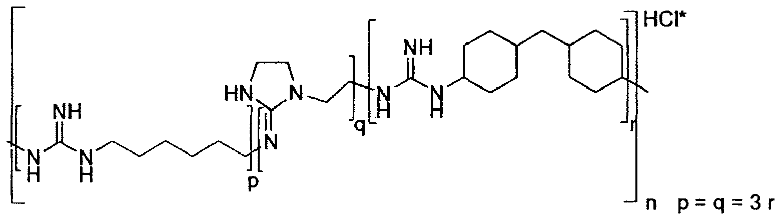 où
HCl* signifie que le HCl n'est pas lié de manière covalente,
n est un entier naturel, de préférence de 1 à 20, plus préférablement de 2 à 16, notamment de 3 à 8,
p, q et r sont des entiers définissant le rapport molaire mutuel préféré des fragments structuraux dans les formules.

10. Utilisation selon au moins une des revendications 1 à 9, **caractérisée en ce que** ledit article médical est choisi dans le groupe consistant en des cathéters veineux central, des cathéters veineux périphérique, des tubes respiratoires, des endoprothèses, des produits destinés à être appliqués dans l'anesthésie régionale, notamment des cathéters, des couplages, des filtres ; des produits destinés à la thérapie par infusion, notamment des récipients, des prises, des systèmes de transition, des filtres ; des accessoires, comme des connecteurs, des aiguilles, des valves, des robinets à trois voies, des seringues, des conduits, des ouvertures à injection ; des produits de formulation, notamment des ensembles de transfert, des ensembles de mixage ; des produits urologiques, notamment des cathéters, des dispositifs de mesure et de collection d'urine ; des drains de plaie ; des pansements pour plaies ; des matériaux de suture chirurgicale ; des accessoires d'implantation et des implants, notamment des implants en matière plastique, par exemple, des bandages herniaires, des non-tissés, des tricots, des tissus de mailles, des prises, des cathéters à chambre implantables, des prothèses vasculaires ; des désinfectants ; des instruments chirurgicaux jetables ; des drains thoraciques ; des sondes ; des cathéters ; des boîtiers pour dispositifs médicaux, notamment pour les pompes à infusion, les dispositifs de dialyse et les écrans, des dentures artificielles ; des récipients pour liquides, notamment des étuis pour lentilles de contact ; des produits de nettoyage et des désinfectants.

11. Procédé pour la production d'un article médical, comprenant les étapes consistant à :
a) combiner et mélanger le produit de liaison biocide tel que défini dans au moins une des revendications 1 à 9 avec éventuellement un polymère thermoplastique, et
b) soumettre le mélange obtenu dans l'étape a) dans un ou plusieurs procédés de façonnage pour former un article médical.

12. Procédé selon la revendication 11, **caractérisé en ce que** ledit produit de liaison biocide est soumis audit procédé de façonnage dans l'étape b) sous forme de granules ou de mélange-maître.

13. Procédé selon la revendication 11 ou 12, **caractérisé en ce que** ledit procédé de façonnage est une extrusion.

14. Article médical, comprenant un produit de liaison biocide tel que défini dans au moins une des revendications 1 à 9.

15. Article médical selon la revendication 14, **caractérisé en ce qu'**il s'agit d'un article médical tubulaire, notamment d'un cathéter.
